(19) Europäisches Patentamt
European Patent Office
Office européen des brevets

(11) EP 2 562 249 A1

(12) EUROPEAN PATENT APPLICATION

(43) Date of publication:
27.02.2013 Bulletin 2013/09

(21) Application number: 11178713.1

(22) Date of filing: 24.08.2011

(51) Int Cl.:
C12N 9/02 (2006.01) C12N 9/90 (2006.01)
C12P 7/46 (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Helmholtz-Zentrum für Infektionsforschung GmbH
38124 Braunschweig (DE)

(72) Inventors:
- Van Duuren, Jozef
38124 Braunschweig (DE)
- Eggink, Gerrit
38124 Braunschweig (DE)
- Karge, Bianka
38124 Braunschweig (DE)
- Dos Santos, Vitor
38124 Braunschweig (DE)

(74) Representative: Kröncke, Rolf et al
Gramm, Lins & Partner GbR
Freundallee 13 a
30173 Hannover (DE)

# (54) Engineered host cells and methods for the production of cis, cis-muconic acid

(57) The present invention relates in a first aspect to an engineered host cell for the production of cis, cis-muconic acid from catechol. In another aspect, the present invention relates to specific pseudomonas strains wherein at least parts of the cat operon are silenced, in particular, wherein the catB gene is silenced. In a further aspect, the present invention relates to a method for production of cis, cis-muconic acid using the engineered host cells or the pseudomonas strains according to the present invention.

EP 2 562 249 A1

SEQUENCE LISTING

<110> Helmholtz-Zentrum fuer Infektinsforschung GmbH

<120> Engineered host cells and methods for the production of cis, cis muconic acid

<130> 3275-031_EP-1

<160> 8

<170> PatentIn version 3.5

<210> 1
<211> 915
<212> DNA
<213> Pseudomonas putida

<400> 1
```
atgaccgtga acatttccca tactgccgag gtacagcagt tcttcgagca ggccgcaggc      60

ttttgtaatg cggccggcaa cccacgcctc aaacgcatcg tgcagcgcct gctgcaggat     120

accgcgcggc tgatcgaaga cctggacatc agcgaagacg agttctggca cgccgtcgat     180

tacctcaacc gcctgggcgg tcgcggcgaa gccgggttgc tggtggcggg gctgggcatc     240

gaacacttcc tcgacctgct gcaggatgcc aaggaccagg aggcagggcg cgttggcggc     300

accccacgca ccatcgaagg cccgttgtac gtggctggcg caccgattgc ccaaggtgaa     360

gtgcgcatgg acgacggcag cgaggagggc gtggccacgg tgatgttcct ggaaggccag     420

gtgctggacc cgcacggacg cccgctgccg ggtgccacgg tcgacctgtg gcatgccaat     480

acccgtggta cctactcgtt cttcgaccaa agccagtcgg cgtacaacct gcgtcggcgc     540

atcgttaccg atgcccaggg gcgctaccgc gcgcgctcca tcgtgccatc gggctatggc     600

tgcgacccgc aggggccaac ccaggaatgc ctggacctgc tgggccgtca tggccagcgc     660

ccggcgcacg tgcacttctt tatctcggcc ccagggtacc ggcacctgac cacgcagata     720

aacctgtcgg gggacaagta cctgtgggat gactttgcct atgccacacg ggatgggctg     780

gtcggggagg tggtgttcgt cgaagggccg gatggtcggc atgccgagct gaagttcgac     840

ttccagttgc agcaggccca gggcggtgcc gatgagcagc gcagcgggcg gccgcgagct     900

ttgcaggagg cctga                                                      915
```

<210> 2
<211> 304
<212> PRT
<213> Pseudomonas putida

<400> 2

```
Met Thr Val Asn Ile Ser His Thr Ala Glu Val Gln Gln Phe Phe Glu
1               5                   10                  15


Gln Ala Ala Gly Phe Cys Asn Ala Ala Gly Asn Pro Arg Leu Lys Arg
            20                  25                  30


Ile Val Gln Arg Leu Leu Gln Asp Thr Ala Arg Leu Ile Glu Asp Leu
```

35 40 . 45

Asp Ile Ser Glu Asp Glu Phe Trp His Ala Val Asp Tyr Leu Asn Arg
50 55 60

Leu Gly Gly Arg Gly Glu Ala Gly Leu Leu Val Ala Gly Leu Gly Ile
65 70 75 80

Glu His Phe Leu Asp Leu Leu Gln Asp Ala Lys Asp Gln Glu Ala Gly
85 90 95

Arg Val Gly Gly Thr Pro Arg Thr Ile Glu Gly Pro Leu Tyr Val Ala
100 105 110

Gly Ala Pro Ile Ala Gln Gly Glu Val Arg Met Asp Asp Gly Ser Glu
115 120 125

Glu Gly Val Ala Thr Val Met Phe Leu Glu Gly Gln Val Leu Asp Pro
130 135 140

His Gly Arg Pro Leu Pro Gly Ala Thr Val Asp Leu Trp His Ala Asn
145 150 155 160

Thr Arg Gly Thr Tyr Ser Phe Phe Asp Gln Ser Gln Ser Ala Tyr Asn
165 170 175

Leu Arg Arg Arg Ile Val Thr Asp Ala Gln Gly Arg Tyr Arg Ala Arg
180 185 190

Ser Ile Val Pro Ser Gly Tyr Gly Cys Asp Pro Gln Gly Pro Thr Gln
195 200 205

Glu Cys Leu Asp Leu Leu Gly Arg His Gly Gln Arg Pro Ala His Val
210 215 220

His Phe Phe Ile Ser Ala Pro Gly Tyr Arg His Leu Thr Thr Gln Ile
225 230 235 240

Asn Leu Ser Gly Asp Lys Tyr Leu Trp Asp Asp Phe Ala Tyr Ala Thr
245 250 255

Arg Asp Gly Leu Val Gly Glu Val Val Phe Val Glu Gly Pro Asp Gly
260 265 270

Arg His Ala Glu Leu Lys Phe Asp Phe Gln Leu Gln Gln Ala Gln Gly
275 280 285

Gly Ala Asp Glu Gln Arg Ser Gly Arg Pro Arg Ala Leu Gln Glu Ala
290 295 300

<210> 3

<211> 936
<212> DNA
<213> Pseudomonas putida

<400> 3

```
tcagccctcc tgcaacgccc gcggccgatg gcttcgcgcc tcggcgtcag gcgacttggc        60
accctgcaag cggaagtcga atgacagctc ggcaaagcgc tcgccttgca caccgcggtc       120
gcgcgccgcc gccgcatcct cgacaaaacg cagttcgccg atcagcccgt cgcgggtggc       180
ataggcaaag tcgtcccaca ggtacttgtc gccagcaaag ttgatctgcg tggtcaggtg       240
gcggtgcccc ggtgccgaga tgaagaagtg cacgtgcgcc gggcgctggc cgtggcggcc       300
gagcaggtcc aggcattcct gggttgggcc ctgcgggtcg cagccatacc cggacggcac       360
gatcgagcgc gcgcggtagc ggccctcggc atcggtgatg atacgccgac gcaggttgaa       420
ctcggactgg gtcgaatcga agtacgaata ggtgccctgg gtattggcgt gccacaggtc       480
gacggtggca ccggccaacg gcttgccgtc ggcatcgaac acctggccct gaaggaacat       540
caccacgcct gggtcagtgc cgtcgtccat gcgcgcttcg ccctgggcca gcggcgcccc       600
ggcaacgtac aacgggcctt cgatggtgcg cggggtgccg ccgccaaggc cggcttcggc       660
atccttggca tcctgcagca ggtcgaggaa gtgctcgata cccaggccag cagccagcag       720
gcctgcctcg ttacggccgc ccaggcggtt gaggtagtcg acggcgtgcc agaactcgtc       780
ctcggtaatc tccaggtctt cgatcaggcg ggcggtgtct tgcagcacgc gcagaatgat       840
ctgcttgaag cgcgggtttc cttcggcatg gtccaggcca gctacccggt tgaagaaggc       900
ttgaatgtcg gcagtgtggg aaattttcac ggtcat                                 936
```

<210> 4
<211> 311
<212> PRT
<213> Pseudomonas putida

<400> 4

```
Met Thr Val Lys Ile Ser His Thr Ala Asp Ile Gln Ala Phe Phe Asn
1               5                   10                  15

Arg Val Ala Gly Leu Asp His Ala Glu Gly Asn Pro Arg Phe Lys Gln
            20                  25                  30

Ile Ile Leu Arg Val Leu Gln Asp Thr Ala Arg Leu Ile Glu Asp Leu
        35                  40                  45

Glu Ile Thr Glu Asp Glu Phe Trp His Ala Val Asp Tyr Leu Asn Arg
    50                  55                  60

Leu Gly Gly Arg Asn Glu Ala Gly Leu Leu Ala Ala Gly Leu Gly Ile
65                  70                  75                  80

Glu His Phe Leu Asp Leu Leu Gln Asp Ala Lys Asp Ala Glu Ala Gly
                85                  90                  95
```

```
Leu Gly Gly Gly Thr Pro Arg Thr Ile Glu Gly Pro Leu Tyr Val Ala
            100                 105                 110

Gly Ala Pro Leu Ala Gln Gly Glu Ala Arg Met Asp Asp Gly Thr Asp
        115                 120                 125

Pro Gly Val Val Met Phe Leu Gln Gly Gln Val Phe Asp Ala Asp Gly
    130                 135                 140

Lys Pro Leu Ala Gly Ala Thr Val Asp Leu Trp His Ala Asn Thr Gln
145                 150                 155                 160

Gly Thr Tyr Ser Tyr Phe Asp Ser Thr Gln Ser Glu Phe Asn Leu Arg
                165                 170                 175

Arg Arg Ile Ile Thr Asp Ala Glu Gly Arg Tyr Arg Ala Arg Ser Ile
            180                 185                 190

Val Pro Ser Gly Tyr Gly Cys Asp Pro Gln Gly Pro Thr Gln Glu Cys
        195                 200                 205

Leu Asp Leu Leu Gly Arg His Gly Gln Arg Pro Ala His Val His Phe
    210                 215                 220

Phe Ile Ser Ala Pro Gly His Arg His Leu Thr Thr Gln Ile Asn Phe
225                 230                 235                 240

Ala Gly Asp Lys Tyr Leu Trp Asp Asp Phe Ala Tyr Ala Thr Arg Asp
                245                 250                 255

Gly Leu Ile Gly Glu Leu Arg Phe Val Glu Asp Ala Ala Ala Ala Arg
            260                 265                 270

Asp Arg Gly Val Gln Gly Glu Arg Phe Ala Glu Leu Ser Phe Asp Phe
        275                 280                 285

Arg Leu Gln Gly Ala Lys Ser Pro Asp Ala Glu Ala Arg Ser His Arg
    290                 295                 300

Pro Arg Ala Leu Gln Glu Gly
305                 310

<210>  5
<211>  1122
<212>  DNA
<213>  Pseudomonas putida

<400>  5
tcagcgacgg gcgaagcgcg ccaggcgctg ttcgtccaac gtcaggccca ggcctggggt      60

gtgggggatg tgcagctgga agtcgcggta ttgcggcggc tcgttgacga tctcctcggt     120

cagcagcagc ggcccgaaca gctctgtacc ccaggtgagc tggcgcaggg tgaggaatgc     180
```

```
atgagccgaa gccagggtgc cgatcgaacc ttcgagcatg gtgccgccgt acaaggcgat      240
gccagcggcc tcggcgatct gtgcagtgcg cagaaccgcg cgcgggccac cattcttggc      300
gattttcagg gcgaagatgc tggcggcgcc gtcggcggcc aggctgaagg cgtcctcgac      360
gctttcgatc gactcatcgg ccatgatcgg agccggactg cgctggttca ggcgcacctg      420
gccagcgcgg ttgatgcgcg aaatcggctg ctcgatcagg tcgatgccgt tgtcgcccaa      480
tacctggcag gcgcggatgg cctgggactc gtcccagtac tggttgacgt cgacccgcac      540
gctggcgctg tcacccagct cgcgcttgat cgcgaccacg tgcttgaggt cctgcgccac      600
cgggttggcg ccgattttca gcttgaacac gcggtgccgg cgaatgtcca gcatgtgctg      660
tgcttcggcg atgtcgcggg cggtgtcgcc gctggccagg gtccaggcca cttccaggct      720
gtcacgcacg cggccaccca gcagttcgct gaccggcagg cccaggcgtt tgccctgggc      780
gtcgagcaag gcgctttcga tgccggactt ggcgaaggtg ttgcccttgg ccagcttgtc      840
cagcttgagc atggcggcat tgatgttgtc tgccggcagg ccaatcaacg ctggggcgag      900
gtacgcgtcg atgttggcct tgatcccttc ggggctttcg tagccatacg ccaggccacc      960
gatggtggtg gcttcaccga tgccttccac gccatcgctg cagcgcagtc gcaataccac     1020
cagggtctgc tgctgcatgg tgtgcatcgc cagcttgtgc gggcgaatgg tcgggagatc     1080
gacgataatt gcatctatgt gttcaatcag cacgcttgtc at                        1122
```

```
<210>  6
<211>  373
<212>  PRT
<213>  Pseudomonas putida

<400>  6

Met Thr Ser Val Leu Ile Glu His Ile Asp Ala Ile Ile Val Asp Leu
1               5                   10                  15

Pro Thr Ile Arg Pro His Lys Leu Ala Met His Thr Met Gln Gln Gln
            20                  25                  30

Thr Leu Val Val Leu Arg Leu Arg Cys Ser Asp Gly Val Glu Gly Ile
        35                  40                  45

Gly Glu Ala Thr Thr Ile Gly Gly Leu Ala Tyr Gly Tyr Glu Ser Pro
    50                  55                  60

Glu Gly Ile Lys Ala Asn Ile Asp Ala Tyr Leu Ala Pro Ala Leu Ile
65                  70                  75                  80

Gly Leu Pro Ala Asp Asn Ile Asn Ala Ala Met Leu Lys Leu Asp Lys
                85                  90                  95

Leu Ala Lys Gly Asn Thr Phe Ala Lys Ser Gly Ile Glu Ser Ala Leu
            100                 105                 110
```

```
Leu Asp Ala Gln Gly Lys Arg Leu Gly Leu Pro Val Ser Glu Leu Leu
        115                 120                 125

Gly Gly Arg Val Arg Asp Ser Leu Glu Val Ala Trp Thr Leu Ala Ser
    130                 135                 140

Gly Asp Thr Ala Arg Asp Ile Ala Glu Ala Gln His Met Leu Asp Ile
145                 150                 155                 160

Arg Arg His Arg Val Phe Lys Leu Lys Ile Gly Ala Asn Pro Val Ala
                165                 170                 175

Gln Asp Leu Lys His Val Val Ala Ile Lys Arg Glu Leu Gly Asp Ser
            180                 185                 190

Ala Ser Val Arg Val Asp Val Asn Gln Tyr Trp Asp Glu Ser Gln Ala
        195                 200                 205

Ile Arg Ala Cys Gln Val Leu Gly Asp Asn Gly Ile Asp Leu Ile Glu
    210                 215                 220

Gln Pro Ile Ser Arg Ile Asn Arg Ala Gly Gln Val Arg Leu Asn Gln
225                 230                 235                 240

Arg Ser Pro Ala Pro Ile Met Ala Asp Glu Ser Ile Glu Ser Val Glu
                245                 250                 255

Asp Ala Phe Ser Leu Ala Ala Asp Gly Ala Ala Ser Ile Phe Ala Leu
            260                 265                 270

Lys Ile Ala Lys Asn Gly Gly Pro Arg Ala Val Leu Arg Thr Ala Gln
        275                 280                 285

Ile Ala Glu Ala Ala Gly Ile Ala Leu Tyr Gly Gly Thr Met Leu Glu
    290                 295                 300

Gly Ser Ile Gly Thr Leu Ala Ser Ala His Ala Phe Leu Thr Leu Arg
305                 310                 315                 320

Gln Leu Thr Trp Gly Thr Glu Leu Phe Gly Pro Leu Leu Leu Thr Glu
                325                 330                 335

Glu Ile Val Asn Glu Pro Pro Gln Tyr Arg Asp Phe Gln Leu His Ile
            340                 345                 350

Pro His Thr Pro Gly Leu Gly Leu Thr Leu Asp Glu Gln Arg Leu Ala
        355                 360                 365

Arg Phe Ala Arg Arg
    370
```

```
<210>  7
<211>  33
<212>  DNA
<213>  artificial

<220>
<223>  primer

<400>  7
aaaaacatat gaccgtgaac atttcccata ctg                                33


<210>  8
<211>  31
<212>  DNA
<213>  artificial

<220>
<223>  primer

<400>  8
tttttctcga gtcaggcctc ctgcaaagct c                                  31
```

## Description

The present invention relates in a first aspect to an engineered host cell for the production of cis, cis-muconic acid from catechol. In another aspect, the present invention relates to specific pseudomonas strains wherein at least parts of the cat operon are silenced, in particular, wherein the catB gene is silenced. In a further aspect, the present invention relates to a method for production of cis, cis-muconic acid using the engineered host cells or the pseudomonas strains according to the present invention.

Prior art

*Pseudomonas putida* KT2440 is a metabolically versatile soil bacterium with significant potential for biotechnological applications, particularly in the areas of bioremediation and biotransformation. The genome of *P. putida* KT2440 has been sequenced and annotated, which facilitates further development of the strain as a biocatalyst (Nelson et al., 2002, Environ. Microbiol. 4, 799-808). Furthermore, *P. putida* KT2440 can be used for the expression of cloned genes and it is the first Gram-negative soil bacterium that has been certified as a safety strain by the Recombinant DNA Advisory Committee.

Like other *P. putida* strains, *P. putida* KT2440 grows with benzoate as a single carbon source (Jiménez et al., 2002, Environ. Microbiol. 4, 824-841). Benzoate is converted to a diol by a co-factor dependent dioxygenase (Fig. 1). The resulting diol is further dehydrogenated to catechol, which regenerates the co-factor. Catechol is degraded to $H_2O$ and $CO_2$ via the so-called *ortho*-cleavage pathway and the Krebs cycle. The ring cleavage of catechol is carried out by a cofactor-independent catechol 1,2-dioxygenase, which converts catechol to cis, cis-muconic acid or cis, cis-muconate, respectively. The benzoate pathway is encoded by genes that belong to the *ben, cat* and *pca* operons (Fig. 1). *P. putida* KT2440 may have a functional overlap at the level of catechol 1,2-dioxygenase, since the genes PP_3166 *(catA2)* on the *ben* operon and *catA* on the cat operon have a 76 % identity in the amino acid sequence. The extra *catA* gene has not been found in other *ben* operons of *Pseudomonas* species (Jiménez et al., 2002). The expression and physiological function of the *catA2* gene has not been described yet. Ring cleavage of catechol has been described as a rate limiting step in the degradation of aromatic compounds (Kim et al., 1988, Biotechnol. Bioprocess Eng. 3, 112-114).

Intermediates of the benzoate pathway, such as benzoate diol and *cis, cis*-muconate, contain functional groups and may be of interest to the chemical industry. Benzoate diol can be highly functionalized by oxidative and rearrangement reactions and *cis, cis*-muconate contains a dicarboxylic acid structure with conjugated double bonds, which can be hydrogenated to form adipic acid, a raw material for nylon-6,6. Due to the complexity of the conversion process and the involvement of a cofactor-dependent dioxygenase, whole cell biocatalysis is likely to be more suitable to produce compounds of industrial interest from benzoate than *in vitro* biocatalysis with purified enzymes. Furthermore, oxygenases are generally more stable *in vivo.*

Adipic acid is a six-carbon organic molecule that is a chemical intermediate in manufacturing processes used to make certain polyamides, polyurethanes and plasticizers, all of which have wide applications in producing items such as carpets, coatings, adhesives, elastomers, food packaging and lubricants, for example. Some large-scale processes for making adipic acid include i) liquid phase oxidation of ketone alcohol oil (KA oil); ii) air oxidation/hydration of cyclohexane with boric acid to make cyclohexanol, followed by oxidation with nitric acid; and iii) hydrocyanation of butadiene to a pentenenitrile mixture, followed by hydroisomerization of adiponitrile, followed by hydrogenation. Each of the latter processes requires use of noxious chemicals and/or solvents, some require high temperatures, and all require significant energy input. In addition, some of the processes emit toxic byproducts (such as nitrous oxide) and give rise to environmental concerns.

Provided herein are methods for producing adipic acid and other organic chemical intermediates using biological systems. Such production systems may have significantly less environmental impact and could be economically competitive with current manufacturing systems. Thus provided herein are methods for manufacturing adipic acid by engineered host cells.

The ability to produce cis, cis-muconic acid/ cis, cis-muconate from benzoate has been reported for several microorganisms: *Pseudomonas sp.* B13 (Schmidt, E., Knackmuss, HJ., 1984, Apl. Microbiol. Biotechnol. 20, 351-355), *P. Putida* BM014 (Bang S.G., Choi S.Y., 1995, J. Ferment. Bioeng. 79:381-383), *Atrobacta sp.* (Mizuno S., et.al., 1988, Apl. Microbiol. Biotechnol). 28, 20-25 and *Sphingobakterium sp.* GCG (Wu C.M., et.al., 2006, Biochem. Eng. J. 29, 35-40).

Since cis, cis-muconate represents an intermediate compound being of interest to the chemical industry, e.g. in the production of adepic acid, the raw material for nylon-6,6, new production methods and production pathways are desired. In particular, it is of interest to provide new biological based processes and biocatalysts to produce cis, cis-muconic acid/ cis, cis-muconate.

Brief description of the present invention

The present inventors aim to provide new engineered host cells for the production of cis, cis-muconic acid from benzoate via catechol. The engineered host cells are characterised in comprising a foreign gene or heterologeous gene of SEQ ID No. 1 or derivatives thereof encoding for a polypeptide of SEQ ID No. 2 having a catechol 1,2-dioxygenase activity. The polypeptide of SEQ ID No. 2 corresponds to the protein encoded by the catA2 gene also known as PP_ 3166 gene identified in P. putida KT2440. The gene reference number of said protein is AAN68774 and the NCBI reference sequence is NP_745310. The gene symbol is PP_3166.

In another aspect, the present invention relates to a *pseudomonas* strain wherein the catB gene is silenced, preferably knocked-down or knocked-out, or deleted from the chromosome of the *pseudomonas* strain.

In another aspect, the present invention relates to a method for the production of cis, cis-muconic acid comprising the step of culturing the engineered host cell according to the present invention or the *pseudomonas* strain according to the present invention under culture conditions in which the cultured host cell or *pseudomonas* strain produces cis, cis-muconic acid.

Finally, the present invention relates to a method for the generation of host cells suitable for the production of cis, cis-muconic acid from benzoate via the intermediate catechol comprising the step of introducing a foreign gene of SEQ ID No. 1 or derivates thereof encoding for a polypeptide of SEQ ID No. 2 having a catechol 1,2-dioxygenase activity into the host cell.

Brief description of the drawings

Fig. 1 Metabolic pathway of benzoate and the genetic organisation of the ben, cat and pca operons on the genome of P. putida KT2440 (Jiménez et.al., 2002, Environ. Microbiol. 4,824-841).

Detailed description of the present invention

The present inventors identified that the gene PP_3166 also described as catA2 gene of *Pseudomonas putida* KT2440 is a powerful catechol 1,2-dioxygenase activity. In particular, it has been identified that the catA2 gene encoding the catechol 1,2-dioxygenase allows to produce cis, cis-muconic acid from catechol in very high amounts. The specific production rate *($q_{pm}$* is at least eight times higher than the qpm-values reported for other cis, cis-muconate producing strain (e.g. Schmidt and Knackmuss 1984). Furthermore, the catA2 enzyme has a $K_M$ of 0.0010 mM catechol and a $IC_{50}$ of 0.1392 mM cic, cis-muconate at a concentration of 0.05 mM catechol. A $K_i$ value of 0.0028 mM was calculated as detailed below. The $V_{max}$ is estimated to be 0.4 μM/min/mg. Hence, cells containing said genes represent a powerful biocatalyst for the production of cis, cis-muconic acid/ cis, cis-muconate from catechol.

Hence, in a first aspect, the present invention relates to an engineered host cell for the production of cis, cis-muconic acid from catechol which host cell comprises a foreign gene of SEQ ID No. 1 or derivatives thereof encoding for a polypeptide of SEQ ID No. 2 having a catechol 1,2-dioxygenase activity.

As used herein, the term "foreign gene" refers to a gene or a part of a gene which is not present in e.g. the chromosome of the host cell.

The term "foreign gene or heterologous gene" are used herein interchangeably.

The term "SEQ ID No. 1 or derivates thereof" refers to the gene of SEQ ID No. 1. In addition, "derivatives thereof" are meant to include nucleic acid sequences wherein the sequence differ from SEQ ID No. 1 insofar that the coding sequence encodes the same polypeptide but may use different codons due to the degenerative code. In addition, the term "derivatives thereof" include nucleic acid sequences wherein single codons are amended to consider the different codon usage in different organisms.

The term "engineered host cell" as used herein refers to a modified host cell that includes one or more activities distinct from an activity present in a host cell utilized as a starting point (hereafter a "parent host cell"). An engineered host cell includes a heterologous polynucleotide in some embodiments, and in certain embodiments, an engineered host cell has been subjected to selective conditions that alter an activity, or introduce an activity, relative to the parent host cell. Thus, an engineered host cell has been altered directly or indirectly by a human being. A parent host cell sometimes is a native host cell, and at times is a host cell that has been engineered to a certain point.

It is preferred that the engineered host cell according to the present invention is a host cell, in particular, a bacterial strain. In another preferred embodiment, the engineered host cell may be a eukaryotic cell, in particular, a yeast cell.

In some embodiments an engineered host cell is a single cell organism, often capable of dividing and proliferating. A host cell can include one or more of the following features: aerobe, anaerobe, filamentous, non-filamentous, monoploid, dipoid, auxotrophic and/or non- auxotrophic. In certain embodiments, an engineered host cell is a prokaryotic host cell (e.g., bacterium), and in certain embodiments, an engineered host cell is a non-prokaryotic host cell. In some embodiments, an engineered host cell is a eukaryotic host cell (e.g., yeast, fungi, amoeba).

Any suitable yeast may be selected as a parent host cell, engineered host cell or source for a heterologous polynucleotide. Yeast include, but are not limited to, Yarrowia yeast(e.g., Y. lipolytica (formerly classified as Candida lipolytica)), Candida yeast (e.g., C. revkaufi, C. pulcherrima, C. tropicalis, C. utilis), Rhodotorula yeast (e.g., R. glutinus, R. graminis), Rhodosporidium yeast (e.g., R. toruloides), Saccharomyces yeast (e.g., S. cerevisiae, S. bayanus, S. pastorianus, S. carlsbergensis), Cryptococcus yeast, Trichosporon yeast (e.g., T. pullans, T. cutaneum), Pichia yeast (e.g., P. pastoris) and Lipomyces yeast (e.g., L. starkeyii, L. lipoferus).

Any suitable fungus may be selected as a parent host cell, engineered host cell or source for a heterologous polynucleotide. Non-limiting examples of fungi include, but are not limited to, Aspergillus fungi (e.g., A. parasiticus, A. nidulans), Thraustochytrium fungi, Schizochytrium fungi and Rhizopus fungi (e.g., R. arrhizus, R. oryzae, R. nigricans). In some embodiments, a fungus is an A. parasiticus strain that includes, but is not limited to, strain ATCC24690, and in certain embodiments, a fungus is an A. nidulans strain that includes, but is not limited to, strain ATCC38163.

Any suitable prokaryote may be selected as a parent host cell, engineered host cell or source for a heterologous polynucleotide. A Gram negative or Gram positive bacteria may be selected. Examples of bacteria include, but are not limited to, Bacillus bacteria (e.g., B. subtilis, B. megaterium), Acinetobacter bacteria, Norcardia baceteria, Xanthobacter bacteria, Escherichia bacteria (e.g., E. coli (e.g., strains DH10B, Stbl2, DH5-alpha, DB3, DB3.1 ), DB4, DB5, JDP682 and ccdA-over (e.g., U.S. Application No. 09/518,188))), Streptomyces bacteria, Erwinia bacteria, Klebsiella bacteria, Serratia bacteria (e.g., S. marcessans), Pseudomonas bacteria (e.g., P. aeruginosa), Salmonella bacteria (e.g., S. typhimurium, S. typhi), Megasphaera bacteria (e.g., Megasphaera elsdenii). Bacteria also include, but are not limited to, photosynthetic bacteria (e.g., green non-sulfur bacteria (e.g., Choroflexus bacteria (e.g., C. aurantiacus), Chloronema bacteria (e.g., C. gigateum)), green sulfur bacteria (e.g., Chlorobium bacteria (e.g., C. limicola), Pelodictyon bacteria (e.g., P. luteolum), purple sulfur bacteria (e.g., Chromatium bacteria (e.g., C. okenii)), and purple non-sulfur bacteria (e.g., Rhodospirillum bacteria (e.g., R. rubrum), Rhodobacter bacteria (e.g., R. sphaeroides, R. capsulatus), and Rhodomicrobium bacteria (e.g., R. vanellii)).

Cells from non-microbial organisms can be utilized as a parent host cell, engineered host cell or source for a heterologous polynucleotide. Examples of such cells, include, but are not limited to, insect cells (e.g., Drosophila (e.g., D. melanogaster), Spodoptera (e.g., S. frugiperda Sf9 or Sf21 cells) and Trichoplusa (e.g., High-Five cells); nematode cells (e.g., C. elegans cells); avian cells; amphibian cells (e.g., Xenopus laevis cells); reptilian cells; and mammalian cells (e.g., NIH3T3, 293, CHO, COS, VERO, C127, BHK, Per-C6, Bowes melanoma and HeLa cells). Cells used as parent host cells or source for a heterologous polynucleotide are commercially available. Host cells and cells described herein, and other suitable cells are available, for example, from Invitrogen Corporation, (Carlsbad, CA), American Type Culture Collection (Manassas, Virginia), and Agricultural Research Culture Collection (NRRL; Peoria, Illinois).

Parent host cells and engineered host cells may be provided in any suitable form. For example, such host cells may be provided in liquid culture or solid culture (e.g., agar-based medium), which may be a primary culture or may have been passaged (e.g., diluted and cultured) one or more times. Host cells also may be provided in frozen form or dry form (e.g., lyophilized). Host cells may be provided at any suitable concentration.

That is, the term "engineered host cell" refers to host cells being genetically engineered by introducing a foreign or heterologous gene into the cells. The gene may be present in the chromosome of said host cell or may be present on extrachromosomal elements, like plasmids.

With the term "silenced" is meant that the expression of the gene is suppressed or inhibited. The skilled person is well aware of suitable methods for silencing endogenous genes, e.g. by manipulating the promoter region at a gene. It is preferred that the endogenous gene is knocked-down or knocked-out using by way of known methods, e.g. by recombinase techniques. Alternatively, the endogenous gene may be deleted from the chromosome by allelic substitution etc.

For example, the catB gene may be silenced by manipulating e.g. deleting catR in the cat operon. In addition, the catB gene may be silenced by manipulating the sole gene or by manipulating the whole cat operon.

The term "benA" refers to a benzoate dioxygenase, alpha-subunit.

The term "benB" refers to a benzoate dioxygenase, beta-subunit.

The term "benC" refers to a oxidoreductase FAD/NAD (P)-binding domain protein.

The term "benD" refers to the 1,6-dihydroxycyclohexa-2,4-diene-1-carboxylate dehydrogenase.

The term "catA" or "catA1 " refers to the catechol 1,2-dioxygenase, e.g. of SEQ ID No. 3 and 4.

The term "catA2" refers to the catechol 1,2-dioxygenase of SEQ ID No. 1 and derivatives thereof and 2.

The term "catB" refers to the muconate and chloromuconate cycloisomerase. The catB gene is exemplified by SEQ ID No. 5 encoding the catB enzyme of SEQ ID No. 6.

According to the present invention, it is preferred that the genes encoding for enzymes responsible for the processing of the cis, cis-muconic acid/ cis, cis-muconate is silenced, preferably knocked-down or knocked-out, or deleted from the host cell, e.g. from the chromosome of the host cell. Thus, the host cell in which the desired product, namely, cis, cis-muconic acid/ cis, cis-muconate accordingly is not in a position to process the cis, cis-muconic acid further. In this connection, the terms "cis, cis-muconic acid" and "cis, cis-muconate" are used interchangeably herein.

In a preferred embodiment, the engineered host cell according to the present invention is a host cell wherein the endogenous gene encoding for the catechol 1,2-dioxygenase (catA) and/or the catB gene of the host cell or homologs thereof is silenced, preferably knocked-down or knocked-out, or deleted from e.g. chromosome of the host cell.

With the term "analogs of the catechol 1,2-dioxygenase (catA) and/or the catB gene" is meant the gene encoding for a catechol 1,2-dioxygenase or a muconate and chloromuconatecycloisomerase.

In another preferred embodiment, the engineered host cell according to the present invention comprises additional foreign genes encoding for polypeptides having an enzymatic activity useful in the metabolic pathway from the conversion of benzoate via catechol to cis, cis-muconic acid. In particular, it is preferred that said genes encode for the benzoatedioxygenase, alpha-subunit, benzoatedioxygenase, beta-unit, oxidoreductase FAD/NAD (P)-binding domain protein, and/or 1,6-dihydroxycyclohexa-2,4-diene-1-carboxylatedehydrogenase. In particular, the genes are selected from benA, benB, benC or benD.

In this connection, it is noted that the term "genetic engineering", "engineering" or "genetic modification" refers to a suitable nucleic acid addition, removal or alteration that facilitates production of a target product in the engineered host cell. Genetic modification includes, without limitation, insertion of one or more nucleotides in a native nucleic acid of a parent host cell in one or more locations, deletions of one or more nucleotides in a native nucleic acid of a parent host cell in one or more locations, modification or substitution of one or more nucleotides in a native nucleic acid of a parent host cell in one or more location, insertion of a non-native nucleic acid into a parent host cell (e.g. insertion of nucleic acid sequences, etc.).

In another aspect, the present invention relates to a *pseudomonas* strain wherein the catB gene is silenced, preferably knocked-down or knocked-out, or deleted from the chromosome. An example of said *pseudomonas* strain is the pseudomonas strain derived from *P. Putida* KT2440, namely *P. Putida* KT2440-JDI. As described below, said derivative, *P. Putida* KT2440-JDI, is characterised in having a silenced or mutated catR in the cat operon.

The *pseudomonas* strain is particularly a *pseudomonas* strain wherein the catB gene is a gene of SEQ ID No. 5 or derivatives thereof encoding for a polypeptide of SEQ ID No. 6.

In addition, it is preferred that the *pseudomonas* strain being a mutant *pseudomonas* strain being catR deficient. That is, the cat operon is altered by genetic mutation, thus, the transcription thereof is silenced.

The culture medium for cultivation and production of cis, cis-muconic acid contains a carbon source. The skilled person is well aware of suitable carbon sources. Preferred carbon sources include saccinate, citrate or glucose.

In another aspect, the present invention relates to a method for the production of cis, cis-muconic acid. Said method comprises the step of culturing an engineered host cell according to the present invention or a *pseudomonas* strain according to the present invention under culture conditions in which the cultured host cell or *pseudomonas* strain produces cis, cis-muconic acid. It is preferred tat the culture medium comprises benzoate and/or glucose. It is particular prefreed that the benzoate is present in an amount of at least 1 mmol but preferably not higher than 50 mmol.

The method according to the present invention is particularly useful for the production of cis, cis-muconic acid as an intermediate in the production of adipic acid. When producing adipic acid, the cis, cis-muconic acid is produced according to the method of the present invention, and, in addition, the intermediate cis, cis-muconic acid is further hydrogenated for conversion to adipic acid by known methods.

The cis, cis-muconic acid is preferably enriched or isolated from the supernatant and/or the biomass of the host cells.

In this connection, it has been recognised by the present inventors that overdosing benzoate as starting material should be avoided since overdosing may inhibit production of cis, cis-muconic acid. In addition, it is favourable that the cis, cis-muconic acid is permanently removed from the culture to avoid any inhibition by cis, cis-muconic acid. Moreover, the level of catechol in the culture should be kept at a low level to avoid any negative influence of catechol on the culture. Consequently, it is preferred that the method includes a pH-stat-fed-batch process to enhance the production of cis, cis-muconate from benzoate by the engineered host cells or the pseudomonas strains according to the present invention, as described in the following:

**pH-stat fed-batch process.** When *P. putida* KT2440-JD1, a strain described below, is applied for the production of *cis, cis*-muconate, it is important to prevent its exposure to high concentrations of benzoate, since it reduces the $\mu_{max}$ and probably leads to the accumulation of catechol, which reduces the expression of the *ben* operon and may be toxic to *P. putida* KT2440-JD1 since catechol generates reactive oxygen species and spontaneously forms coloured polymers.

Since the medium is acidified when benzoate is converted to *cis, cis*-muconate, it is possible to prevent the accumulation of benzoate by coupling the addition of benzoate to the pH-regulation. Therefore, a pH-stat fed-batch process was designed in which the addition of benzoic acid was coupled to the pH-regulation with NaOH at a benzoic acid:NaOH molar ratio of 1:2. For this, a bioreactor containing MM with 10 mM of glucose was inoculated with *P. putida* KT2440-JD1. 1 mM of benzoate was added after 1 hour to induce the cells. Continuous addition of glucose started when glucose was depleted. The feeding rate of glucose was increased every hour to obtain exponential feeding, which resulted in a growth rate of 0.04 $h^{-1}$. During this period the glucose concentration never exceeded 0.3 mM. After 13 hours, production of *cis, cis*-muconate was started by adding 20 mM benzoate to the culture to maximally induce the cells. The pH was maintained at 7.0 by the automated addition of 10 M of NaOH. Catechol accumulated in the culture up to 6 mM but disappeared within 6 h. The temporary presence of catechol confirmed that catechol accumulates in the culture medium when benzoate is present in high concentrations. When benzoate was almost completely converted to *cis, cis*-muconate (as determined by HPLC), the feeding of benzoate was coupled to the acidification of the culture by titrating the culture to pH 7.5 with a solution of 1.2 M benzoic acid and 2.4 M NaOH as soon as the pH became lower than 7.0. In this way, the concentrations of benzoate and catechol were kept low.

After the pulse of 20 mM benzoate, the $qp_m$ increased to a maximum value of 0.6 g (4.3 mmol) g $dcw^{-1}$ $h^{-1}$, which resulted in a maximal volumetric productivity of 0.8 g $L^{-1}$ $h^{-1}$. The $q_{pm}$ gradually decreased during the accumulation of *cis, cis*-muconate and completely stopped at a concentration of 18.5 g $L^{-1}$ (130 mM). The overall molar product yield $(C_{pm}\,C_{Sb}^{-1})$ was close to 100 %. The production of biomass continued when the production of *cis, cis*-muconate became completely inhibited, indicating that *P. putida* KT2440-JD1 was still able to grow at this concentration of *cis, cis*-muconate. Extra benzoate was added to stimulate *cis*, *cis*-muconate production, but the concentration of *cis, cis*-muconate did not increase. Instead, benzoate, catechol, and glucose accumulated in the medium and growth of *P. putida* KT2440-JD1 stopped. Due to the conversion of benzoate to catechol, the pH of the medium increased from 7.5 to 8.5.

Various processes have been reported for the accumulation of *cis, cis*-muconate from benzoate using aerobic bacteria (Table 3). The exposure of the culture to high concentrations of benzoate was avoided by feeding under controlled conditions at a low constant rate or in pulses, or by coupling the addition of benzoate to the depletion of oxygen (DO-stat) or the decrease of the pH (pH-stat). The advantage of a pH-stat compared to a DO-stat is the decoupling of the feeding of benzoate from the feeding of the growth substrate (glucose), because benzoate is only replenished when the medium acidifies by its own conversion to *cis, cis*-muconate.

**Table 3**. Maximum specific productivities $(q_{pm})$, maximum volumetric productivities and final concentrations of *cis,* cis-muconate of various production processes of *cis, cis*-muconate from benzoate using different strains.

| Process | Strain | Maximum $q_{pm}$ (g g $dcw^{-1}$ $h^{-1}$) | Maximum volumetric productivity (g $L^{-1}$ $h^{-1}$) | Final concentration (g $L^{-1}$) |
|---|---|---|---|---|
| pH-stat fed-batch | *P. putida* KT2440-JD1 (this research) | 0.60 | 0.8 | 18.5 |
| fed-batch | *Spingobacterium* sp. GCG[7] | >0.10 | >0.1 | >0.1 |
| cell-recycle system | *P. putida BM0*14[5] | 0.14** | 5.5** | 12.0** |
| DO-stat fed-batch | *P. putida* BM014[4] | 0.21 | 2.2 | 32.0 |
| fed-batch | *Arthrobacter* sp.[6] | n.d.* | 1.1 | 44.0 |
| fed-batch | *Pseudomonas* sp. B13[3] | 0.24 | 0.8 | 7.0 |

not determined; steady values.

3. Schmidt E, Knackmuss HJ. 1984, Appl Microbiol Biotechnol 20:351-355.
4. Bang SG, Choi CY. 1995, J Ferment Bioeng 79:381-383.
5. Choi WJ, Lee EY, Cho MH, Choi CY. 1997, J Ferment Bioeng 84:70-76.
6. Mizuno S, Yoshikawa N, Schi M, Mikawa T, Imada Y. 1988, Appl Microbiol Biotechnol 28:20-25.

The highest volumetric productivity of 5.5 g *cis*, *cis*-muconate $L^{-1}h^{-1}$ was obtained in a system which used a constant inflow rate of benzoate in combination with a membrane to retain the cells, resulting in a culture with a high cell density of 40 g $L^{-1}$. This productivity could be maintained during the whole process and yielded a fermentation effluent of 12 g *cis*, *cis*-muconate $L^{-1}$. An *Arthrobacter sp.* was able to accumulate the highest concentration of *cis, cis*-muconate of 44 g $L^{-1}$ during a fed-batch process, but the $q_{pm}$ was not determined (Table 3). During the pH-stat fed-batch process the highest $q_{pm}$ *was* observed soon after the initial pulse of 20 mM of benzoate, but the productivity was lower than the maximum rates that were previously observed under $\mu_{max}$ conditions[1], and the $q_{pm}$ decreased during the pH-stat process. The $q_{pm}$ *was* also much lower than the $q_{sb}$ that was determined in this study. The discrepancy between the $q_{sb}$ and the $q_{pm}$ is probably caused by the accumulation of the metabolic intermediate catechol, which was formed during the pH-stat fed-batch process, especially after the pulse of 20 mM benzoate. The conversion of catechol can be rate limiting, and over-expression of catechol 1,2-dioxygenase in *P. putida* BCM114 resulted an increased $q_{pm}$ from benzoate. Catechol down-regulates the expression of the *ben* operon in *P. putida* KT2440-JD1, and is known to be a toxic compound.

The $q_{pm}$ of *P. putida* KT2440-JD1 decreased during the pH-stat fed-batch process with increasing concentrations of *cis,* cis-muconate and became completely arrested at 18.5 g $L^{-1}$ *cis, cis*-muconate. At this concentration of *cis*, *cis*-muconate, the growth of *P. putida* KT2440-JD1 and uptake of benzoate was still observed, but catechol started to accumulate in the growth medium instead of *cis, cis*-muconate. The latter may be caused by product inhibition of *cis, cis*-muconate.

Thus, the coupling of the addition of benzoate to the decrease of the pH (pH-stat) proved to be a very suitable method to prevent the overdoses of benzoate to the culture. The process may be further improved by i) enhancing the catechol 1,2-dioxygenase activity in *P. putida* KT2440-JD1 e.g. by genetic engineering; ii) enhancing the volumetric productivity by increasing the $C_x$ in the bioreactor, e.g. by using a cell retention system; iii) preventing the accumulation of *cis, cis*-muconate by an efficient removal of *cis, cis*-muconate. These improvements could lead to a process that optimally exploit the high potential productivity of this strain to convert benzoate to *cis, cis*-muconate.

Finally, the present invention relates to a method for the generation of host cells for the production of cis, cis-muconic acid from catechol comprising the step of introducing a foreign gene of SEQ ID No. 1 or derivatives thereof encoding for a polypeptide of SEQ ID No. 2 having a catechol 1,2-dioxygenase activity, into the host cell. It is particular preferred that the foreign gene is introduced into the genome of a host cell. The skilled person is well aware of suitable methods for introducing the foreign gene into the host cell accordingly.

In the following, the invention will be described by the way of examples without limiting the invention thereon.

<u>Materials and methods</u>

*Strain and cultivation conditions*

*P. putida* KT2440 (DSM6125) and its derivatives were grown in E-2 mineral medium (MM) (Hartmans et al., 1989, Appl. Environ. Microbiol. 55,2850-2855) at 30°C and pH 7. Batch cultivations were done in Erlenmeyer flasks that were shaken at 200 rpm. Continuous cultivations were done in 2 L pH-controlled bioreactors with a working volume of 0.75 L (Applikon, The Netherlands). The oxygen saturation was kept above 50 % by stirring at 550 rpm and sparging with air at 0.75 L/min. Antifoam 204 (Sigma-Aldrich, USA) was added at a concentration of 0.02 %. Samples were taken during steady state, which was assumed to be reached after 5 volume changes. Three independent cultivations were done for each growth condition. After one steady state of the mutant strain *P. putida* KT2440-JD1, which converts benzoate to *cis, cis*-muconate, the dilution rate (D) was increased to 1.3 $h^{-1}$ to cause a wash-out of the biomass. Five samples were taken within 3.5 hours to measure the concentration of biomass *($C_x$)*, benzoate *($C_b$)* and *cis,* cis-muconate *($C_m$)*, from which the maximum growth rate *($\mu_{max}$)* and $q_{pm}$ *were* calculated using the formulas $C_{x_t} = C_{x_o}e^{(\mu-D)t}$ and

$$\frac{dC_{m_t}}{dt} = q_{pm}C_{x_t} - DC_{m_t}.$$

To calculate the $q_{pm}$ this formula was rewritten to:

$$\frac{C_{m_t}}{C_{x_t}} = \frac{q_{pm}}{\mu}(1 - e^{-\mu t}) + \frac{C_{m_0}}{C_{x_0}}e^{-\mu t}.$$

During the steady states and the wash-out experiment the biomass yield *($Y_{(x/S)}$)* ($g_{DCW}$/ g glucose) and the $Y_{(P/S)}$ (mol benzoate/ mol *cis,* cis-muconate) were determined.

The intracellular concentrations of benzoate, catechol and *cis,* cis-muconate were measured in *P. putida* KT2440-JD1, that was grown in an Erlenmeyer shake flask on 35 mL MM with 5 mM benzoate and 10 mM glucose. When benzoate was depleted, the cells were harvested, washed three times with cooled PBS buffer containing 136.9 mM NaCl, 2.68 mM KCl, 4.27 mM $Na_2HPO_4.2H_2O$, 1.47 mM $KH_2PO_4$ (pH 7.13), and frozen at -20ºC. After this, the cells were resuspended and lysed in 1 mL buffer containing 10 mM Tris-HCL and 1 mM EDTA (pH 8) with 1 mg/ml lysozyme at room temperature for 25 min while shaking, after which the concentrations of benzoate, catechol and *cis,* cis-muconate were measured by HPLC.

*Analytical methods*

$C_x$ was determined during steady state by measuring the $OD_{600}$ on a Ultrospec 2000 spectrophotometer (Pharmacia Biotech, Sweden). The relation between $OD_{600}$ and $C_x$ was determined to be $C_x = 0.41 \times OD_{600} + 0.1$ by measuring the $OD_{600}$ of steady state continuous cultures and by weighing the amount of cells in 10 mL culture samples. The latter was done in triplicate by filtering the culture samples through a pre-dried and weighed 0.2 μm cellulose-acetate filter (Whatmann, USA), after which the cells were washed with 5 mL phosphate buffer (3.9 g/L $K_2HPO_4$ and 1.6 g/L $NaH_2PO_4$).

Benzoate and *cis, cis*-muconate were determined in the culture supernatant by HPLC using a Varian Microsorb$^{tm}$-MVHPLC column (length, 250 nm; inside diameter, 4.6 mm; particle size, 5 μm) (Varian, USA) with a mobile phase: 130 mL/L methanol, 130 mL/L acetonitrile, 740 mL/L demineralized water, 26 μL/L TFA and 67.5 μL/L $H_3PO_4$ (85 %) (1.25 mL/min) and a Waters 2487 UV detector (Waters, USA) (Jeffrey et al., 1992, J. Bacteriol. 174,4986-4996). Benzoate was measured at 207 nm and *cis, cis-* muconate at 260 nm. The supernatants were filtered (pore size of 0.2 μm) prior to analysis to remove solids and precipitated proteins.

Glucose in the culture supernatant was determined by HPLC using an Altech IOA-1000 Organic Acids Column (Altech, USA) at 90°C with 3 mM $H_2SO_4$ as the mobile phase (0.4 mL/min) followed by detection using a Waters 2414 differential refractometer (Waters, USA). Prior to analysis, the supernatant was diluted 1:1 with 1 M $H_2SO_4$ and filtered (pore size of 0.2 μm) to remove solids and precipitated proteins.

*NTG-mutagenesis and high throughput screening*

Mutants of *P. putida* KT2440 were generated by exposure to NTG. For this, *P. putida* KT2440 was grown in a batch culture on 4 g/L glucose for two days. The cells were harvested by centrifugation (3,250 × g, room temperature, 25 min) and washed with citrate-phosphate (CPI) buffer: 22.6 g/L $Na_2HPO_4.2H_2O$, 7.6 g/L $C_6H_8O_7.H_2O$. The pH was adjusted to 6.0 with $H_3PO_4$. After washing, the cells were centrifuged and re-suspended in CPI buffer to an $OD_{600}$ of 5 and split in 3 batches of 25 mL. The optimal concentration was determined similar to Adelberg et al., Biochem. Biophys. Res. Commun. 18,788-795 (1965) by adding 0, 60 and 100 μg/mL NTG to the batches. The cells were incubated for 30 min at 30°C, after which the supernatant was discarded by centrifugation. The cells were re-suspended in 25 mL Luria Broth (LB) and incubated for another 30 min at 30°C, after which the cultures were mixed with 12.5 mL 60 % glycerol and frozen at -80°C. The survival rate of the NTG treatment was determined by counting the number of colony forming units (CFU) on 1.5 % agar plates containing MM with 10 mM succinate. Compared to the culture that was not exposed to NTG, the culture that was exposed to 60 ug/ml NTG had a survival rate of 22 %, while the culture that was exposed to 100 μg/ml NTG had a survival rate of 4 *%. P. putida* KT2440 exposed to 60 μg/mL NTG was chosen to continue the research.

*Isolation of mutants that accumulate metabolic intermediates of the benzoate pathway*

The LB cell suspension with the NTG-treated *P. putida* KT2440 was inoculated into 25 mL MM with 20 mM 3-fluorobenzoate (3-FB), 10 mM succinate and 1 mM benzoate to an $OD_{600}$ of 0.1 and grown for 10 hours, after which it was transferred to two flasks with the same medium. One of the cultures was grown for 14 hours and the other for 24 hours. The LB cell suspension and the two cultures were plated on 1.5 % agar plates containing 20 mM 3-FB in MM with 10 mM succinate and 1 mM benzoate. The colonies were selected and inoculated onto 384-well plates containing MM with 10 mM succinate and 1 mM benzoate by using a Versarray® colony arrayer and picker (Bio-Rad, USA). Mutants were screened by testing their ability to grow in MM with either 10 mM benzoate or 10 mM succinate as their only source of carbon and energy. Mutants that grew with succinate, but no longer with benzoate, were frozen at - 80°C in 15 % glycerol.

*Production of cis, cis-muconate during batch cultivations*

*P. putida* KT2440-JD1 was grown in batch cultures in MM with 1 mM benzoate and 10 mM of either succinate, citrate or glucose for 25 hours, after which 2 % of the cultures were transferred to the same medium to determine the $\mu_{max}$ and $q_{pm}$. The $\mu_{max}$ *was* determined from the slope of the natural logarithm of $C_x$ over time during the exponential growth phase. The maximal $q_{pm}$ in the batch cultures was calculated by using the following formula:

$$q_{pm} = \frac{dC_{m_t}}{(\overline{C_{x_t}} \times dt)}.$$

*Transcriptomics*

The transcriptome of each of the *P. putida* strains (KT2440 and KT2440-JD1) were analyzed for two of the three independent steady states that were obtained during continuous cultivation. Samples of 1 mL were taken from the cultures and immediately cooled by adding 1 mL of very cold methanol (-80 4°C). The samples were quickly centrifuged at 16,873 × *g* at 4°C for 50 s, after which the supernatant was discarded. 1 mL RNAlater® (Ambion, USA) was added to stabilize and protect the RNA in the cells. The tubes were vortexed for 1 second and incubated at 4°C for 1-2 h, after which the cells were harvested by centrifugation (16,873 × g, 4°C, 1 min). The cell pellet was dried for 5 min, frozen in liquid nitrogen, and stored at -80 4°C.

Total RNA was isolated from the cell pellet using the Qiagen® RNeasy Mini Purification Kit and OIAGEN®

RNase-Free DNase Set (Qiagen, The Netherlands). The concentration of RNA was determined at 260 nm using a ND-1000 spectrophotometer (NanoDrop, USA). Ten micrograms of total RNA were used to purify 1.2 to 2.2 μg mRNA using the Ambion® MICROB*Express* Bacterial mRNA Purification kit (Ambion, USA). The purity of the isolated RNA was determined using the Bio-Rad Experion System (Bio-Rad, USA). cDNA was generated from mRNA using the Affymetrix Poly-A RNA control kit and random priming (Affymetrix, USA). The concentration of cDNA was determined at 260 nm using a ND-1 000 spectrophotometer. 0.8-1.2 μg cDNA was fragmented with 0.1 U DNaseI (Pharmacia Biotech, Sweden) per μg DNA at 37ºC for 10 min. The fragments were labeled with biotinylated GeneChip® DNA labeling reagent (Affymetrix, USA). The labeled and fragmented cDNA was hybridized to high-density oligonucleotide microarrays of Nimble Express™ arrays (Roche, Switzerland) and scanned by ServiceXS Leiden (Leiden, The Netherlands) according to Ballerstedt et al., Appl. Microbiol. Biotechnol. 75,1133-1142 (2007). The arrays were constructed according to the Affymetrix specifications of *P. putida* A530009N, which was based on the annotated genome of *P. putida* KT2440 (AE015451.1).

The data were analysed using the Bioconductor microarray analysis suite (Gentleman et al., 2004, Genome Biol. 5,R80). Raw data were normalized and the expression values computed with the GC-RMA algorithm using default parameters (Wu et al., 2004, Biochem. Eng. J. 29,35-40). The Rank products algorithm was used to identify differentially expressed genes in pairwise comparisons between two conditions (Breitling et al., 2004, FEBS Lett. 573,83-92). This algorithm identifies such genes by comparing the products of ranks of a particular gene in replicated measurements between the two conditions. It allows for the control of the false discovery rate (FDR - expected fraction of false positives among genes identified as differentially expressed) by providing a parameter termed percentage of false positives (PFP - an estimate of FDR) for each gene and thus addresses the multiple testing problem associated with identification of differences in microarray experiments (simultaneous testing of thousands of genes) (Dudoit et al., 2003, Stat. Sci. 18,71-103). Genes that had a PFP parameter of 0.05 or lower (i.e. 5% or less probability of being false positive) and a fold change above 2 or below 0.5 were considered as differentially expressed.

*Proteomics*

The proteome of each of the three independent steady states of *P. putida* KT2440 and *P. putida* KT2440-JD1 with or without benzoate was analyzed. The culture samples were identical to those used for transcriptomics. Per steady state, seven of these samples were pooled. Proteins were extracted from the pooled samples by using an SDS extraction protocol for *E. coli* (Wijte et al., 2006, J. Proteome. Res. 5,2033-2038) and analyzed by 2-dimensional (2D) gel electrophoresis. Additionally, a master 2D gel was prepared that contained equal amounts of protein from the four different conditions. For the first dimension electrophoresis, 150 μg protein was loaded on a 13 cm immobilized pH gradient (IPG) strip pH 4-7 in 250 μL rehydration buffer (9.5 M urea, 4 % (w/v) 3-[(3-cholamidopropyl)dimethylammonio]-1-propansulfonat, 1 % Destreak (GE Healthcare, USA), pH 8.5). Rehydration and isoelectric focusing was carried out using an IPGphor (GE Healthcare, USA) at 20ºC for a total of 30-57 kVh with the current set to 50 pA per strip. Prior to the second dimension, the IPG strips were incubated for 15 min in equilibration buffer (50 mM Tris-HCl, pH 8.8, 6 M urea, 30 % (v/v) glycerol, 2 % (w/v) SDS) containing 1 % (w/v) dithiothreitol (DTT)), followed by 15 min incubation in equilibration buffer containing 2.5 % (w/v) iodoacetamide instead of DTT. The second dimension electrophoresis was performed on lab-cast 12.5 % polyacrylamide gels in a Hoeffer SE600 electrophoresis unit (Hoeffer, USA). The IPG strips were placed on the top of 12.5 % polyacrylamide gels and sealed with a solution of 1 % (w/v) agarose with a trace of bromophenol blue. Gels were run at 15 mA per gel for 15 min, followed by 35 mA per gel until the bromophenol blue had migrated to the bottom of the gel. Proteins were visualized using Sypro Ruby (SR) (Krieg et al., 2003, Biotechniques 35,376-378) (Bio-Rad, USA). Next, the SR stained gels were scanned using a Molecular Imager FX (Bio-Rad, USA) (100 μm resolution, middle sensitivity, 532 nm laser) and converted with Quantity-One 4.4.0 software (Bio-Rad,USA) to tiff-file format. Post-staining of the SR stained gels was done with ProteomIQ (Proteome Systems, USA) (Wijte et al., 2006, J. Proteome. Res. 5,2033-2038).

Image analysis was performed using PDQuest software version 7.1.0 (Bio-Rad, USA). Normalization of spot volumes in a gel was performed using the 'total density in valid spots' option. The normalized intensity of spots on three replicate 2D gels was averaged. The relative change in protein abundance for *P. putida* strain KT2440 and *P. putida* KT2440-JD1 with or without benzoate was calculated by dividing the averaged normalized spot quantities of the different conditions. Proteins were significantly differentially expressed when the fold change was above 1.5 or below 0.7 and the *p* value was less than or equal to 0.05. Spots of all differently expressed proteins were excised and digested in-gel with trypsin and analyzed with MALDI-MS according to Havlis et al., Anal. Chem. 75,1300-1306 (2003). Peptides were analyzed by the Ultraflex TOF/ TOF Mass Spectrometer (Bruker Daltonics, USA), using delayed extraction and reflectron mode with positive ion detection. The mass scale was calibrated with the trypsin autodigestion product of known $[M+H]^+$ mass, 2211.1 Da and the keratin 9 fragment $[M+H]^+$, 2705.2 Da. Proteins were identified using an in house-licensed Mascot search engine (version 2.1.0, Matrix Science, U.K.). The *P. putida* KT2440 database was searched using the MALDI TOF-MS data with carbamidomethyl cysteine as a fixed modification and oxidized methionine as a variable modification. Trypsin was specified as the proteolytic enzyme and up to two missed cleavages were allowed. The quality of peptide mass fingerprints and the certainty of protein identification were evaluated by MOWSE scores.

*Protein sequence and structure analysis of catechol 1,2-dioxygenases*

Protein sequences of CatA2 (Q88I35), CatA (Q88GK8) and catechol-1,2-dioxygenase from *P. arvilla* C1 (Q51433) were downloaded from Uniprot (htt ://www.uniprit.org) and aligned using ClustalW (Thompson et al., 2002, Curr. Protoc. Bioinformatics. Chapter 2,Unit 2.3). Structural models of the three proteins were downloaded from the SWISS-MODEL Repository (Kiefer et al., 2009, Nucleic Acids Res. 37,D387-392; Kopp and Schwede, 2006, Nucleic Acids Res. 34,D315-318). The structures of CatA and CatA2 are based on the known structure of catechol-1,2-dioxygenase from *P. arvilla* C1 (PDB_Id:2AZQ) (http://www.rcsb.org) (Earhart et al., 2005, Biochem. Biophys. Res. Commun. 338,198-205). The structural models were visually compared using the molecular graphics program Coot the structural models were visually compared (Emsley and Cowtan, 2004, Acta Crystallogr. D Biol Crystallogr. 60,2126-2132).

*Expressing and purification of catA2*

Gene *catA2 was* amplified from the genome of *P. putida* KT2440 by PCR using the forward primer 5'- AAAAACATATGACCGTGAACATTTCCCATACTG-'3 (SEQ ID No. 7) and the reverse primer 5'-TTTTTCTCGAGTCAGGCCTCCTGCAAAGCTC-'3 (SEQ ID No. 8) containing a *Nde*I and *Xho*I site, respectively. The PCR fragment was ligated into the pET-28a(+) expression vector (Novagen, USA). The *catA2* insert was controlled by sequencing after amplification with the T7 and T7 term primer set (Eurofins MWG GmbH, Germany).

pET-28a(+) with and without the *catA2* insert were electro transformed into *E. coli* BLN21 and plated on LB with kanamycin (50 μg/mL). Transformants containing the plasmid with and without *catA2* were grown overnight in 25 mL LB with kanamycin (50 μg/mL) at 37ºC with shaking. 5 mL of these cultures were used to inoculate 200 mL cultures with the same medium, to which 1 mM isopropyl b-d-thio-galactoside was added at the logarithmic phase. The cells were harvested by centrifugation at 13,680 × *g* at 4 4ºC for 30 min and frozen at -20ºC. The frozen cells were resuspended in 15 mL 20 mM Tris buffer (pH 7.5), and lysed by adding a small amount of lysozyme from chicken egg (Fluka, USA) and sonificating. The crude cell extract was centrifuged at 9,047 × *g* at 4 ºC for 10 min. *catA2 was* purified from the crude extract by ÄKTA fast protein liquid chromatography (GE health care, USA) using a 5 mL NI-NTA column (Qiagen, The Netherlands). The proteins were eluted with 20 mM Tris-HCl (pH 8) with a 0-500 mM imidazol gradient at a flow rate of 5 mL/min for 20 minutes. The purification of CatA2 was monitored with SDS-PAGE and Westernblotting using His-Tag antibodies (Abcam, U. K.).

The activity of *catA2* was assayed by measuring the increase in *cis, cis*-muconate concentration from catechol in a buffer containing 20 mM Tris-HCl, 0.125 M imidazole (pH 7.8) at $A_{256}$ using an UV mc2® spectrophotometer (SAFAS, Monaco). In order to determine the $k_m$ value of the enzyme, the conversion rate of catechol to *cis, cis*-muconate was measured at 10 different concentrations of catechol ranging between from 0.0001 to 0.05 mM. The half maximum inhibitory concentration *($IC_{50}$)* was determined from the conversion of 0.05 mM catechol in the presence of 16 different concentrations of *cis, cis*-muconate ranging from 0 to 0.2 mM The protein concentration was determined using the Coomassie plus$^{tm}$ (Bradford) protein assay reagent (Thermo Fischer Scientific, USA). The values for $K_m$, $IC_{50}$, and $V_{max}$ were calculated by Graphpad Prism (Graphpad Software, Inc., USA).

## Results and discussion

*Isolation of P. putida KT2440 mutants that accumulate metabolic intermediates of the benzoate pathway*

*P. putida* KT2440 was exposed to NTG to generate mutants that accumulate benzoate intermediates. To enhance the amount of cells that were mutated in the benzoate pathway, the NTG-treated culture was exposed to 3-FB or 3-chlorobenzoate (3-CIB). Both compounds are substrate analogues of benzoate, and 3-FB is known to become toxic when converted by the benzoate pathway (Schmidt and Knackmuss, 1984, Appl. Microbiol. Biotechnol. 20,351-355). In this way, mutants that do not generate these toxic intermediates will have a selective advantage.

To determine the optimal concentrations of the substrate analogues, *P. putida* KT2440 was grown in MM with 10 mM succinate and 1 mM benzoate in the presence of increasing concentrations of 3-FB or 3-CIB. The accumulation of fluorocatechol derivatives is known to result in a violet coloration of the medium. When *P. putida* KT2440 was exposed to 3-FB or 3-CIB a violet coloration of the medium was observed. Growth of *P. putida* KT2440 was more inhibited and the violet coloration was more intense and rapid when the cells were exposed to 3-FB as compared to 3-CIB. Therefore, two independent batch cultures of NTG-treated *P. putida* KT2440 cells were cultivated in the presence of 20 mM 3-FB. Batch culture 1 was grown for 14 hours and batch culture 2 for 24 hours, after which the cultures were plated. Individual colonies were screened for their ability to grow with benzoate as the sole source of carbon and energy. Three mutants that could no longer grow using benzoate were isolated from batch 1 and 84 mutants were isolated from batch 2, which represented 0.1 % and 4.4 %, respectively, of the total amount of colonies that were screened. No mutants were found when the NTG-treated *P. putida* KT2440 cells were not exposed to 3-FB. All mutants accumulated *cis, cis*-muconate in the medium when grown with succinate in the presence of benzoate. Therefore, the disability of the further conversion of 2-fluoro-*cis*, *cis*-muconate by the muconate lactonizing enzyme seems to have caused the selective advantage. 2-Fluoro-cis, cis-muconate has been observed as a dead-end metabolite (Schmidt and Knackmuss, 1984, Appl. Microbiol. Biotechnol. 20,351-355). One mutant *P. putida* KT2440-JD1 was selected for further study because of its relatively high $Y_{(P/S)}$ in batch culture (data not shown) and because it was isolated from batch 1, which had the shortest period of exposure to 3-FB.

*Conversion of benzoate with different growth substrates*

To determine which carbon and energy sources are suitable for the production of *cis, cis*- muconate from benzoate by *P. putida* KT2440-JD1, the strain was grown in MM with 10 mM of either succinate, citrate, or glucose in combination with 1 mM benzoate. *P. putida* KT2440-JD1 was able to grow well with succinate, citrate or glucose. The $q_{pm}$ and $Y_{(X/S)}$ were higher with glucose, while the $Y_{(P/S)}$ was higher with citrate (Table 1). Since glucose is an inexpensive substrate and the $Y_{(P/S)}$ with glucose was still high, this substrate was chosen for further studies.

*Continuous cultivations of P. putida KT2440 and KT2440-JD1 in the presence and absence of benzoate*

*P. putida* KT2440 and KT2440-JD1 were grown in triplicate in continuous cultures with 10 mM glucose in the presence and absence of 5 mM benzoate at a dilution rate of 0.2 $h^{-1}$. In the absence of benzoate, the $Y_{(X/S)}$ of *P. putida* KT2440 and KT2440-JD1 were 0.37 $\pm$0.02 and 0.36$\pm$0.01 $g_{DCW}$/g glucose, respectively. When 5 mM benzoate was present in the feed of *P. putida* KT2440, the $Y_{(X/S)}$ in steady state cultures increased to 0.58 $\pm$0.04 $g_{DCW}$/g glucose, due to the use of benzoate as additional growth substrate. With P. *putida* KT2440-JD1, the $Y_{(X/S)}$ (0.40 $\pm$0.06 $g_{DCW}$/g glucose) showed no significant change in the presence of benzoate. Benzoate was co-metabolized to *cis,* cis-muconate with an average $Y_{(P/S)}$ of 0.89 $\pm$0.02 mol *cis,* cis-muconate/mol benzoate and a $q_{pm}$ of 0.18 $\pm$0.03 g *cis, cis*- muconate/ ($g_{DCW} \times$ h). The $Y_{(P/S)}$ was similar to that obtained during batch cultivations (Table 1). No benzoate, catechol or *cis,* cis-muconate was detected in the cytosol of P. *putida* KT2440-JD1 that was producing cis, cis-muconate from benzoate during growth on glucose in shake flasks. These results indicate that some *cis, cis*-muconate was further metabolized. When the D was increased to 1.3 $h^{-1}$ after a steady state with *P. putida* KT2440-JD1 in the presence of 5 mM benzoate, the culture washed-out. The $\mu_{max}$ of the mutant during the wash-out was 0.6 $h^{-1}$. The highest $q_{pm}$ *was* determined to be 1.4 g cis, cis-muconate/ ($g_{DCW}\cdot\times$ h) after 1 hour and 20 minutes after the increase of the *D* to 1.3 $h^{-1}$. The $q_{pm}$ was 8 times higher than observed during continuous cultivation at *D* 0.2 $h^{-1}$, showing that the productivity of *P. putida* KT2440-JD1 is much higher under $\mu_{max}$ conditions and unlimited presence of benzoate.

Several other mutants were reported that also convert benzoate to *cis, cis*-muconate (as mentioned before). The $q_{pm}$ of *P. putida* KT2440-JD1 under $\mu_{max}$ conditions was at least 8 times higher than that of the best performing strains *Pseudomonas* sp. B13 (Schmidt and Knackmuss, 1984) and *P. putida* BM014 (Bang and Choi, 1995, J. Ferment. Bioeng. 79,381-383), while the $Y_{(P/S)}$ was similar, showing that *P. putida* KT2440-JD1 is a very suitable biocatalyst for the production of *cis,* cis-muconate.

*Transcriptomics of P. putida KT2440 and KT2440-JD1 in the presence and absence of benzoate*

The transcriptome analysis of *P. putida* KT2440 and KT2440-JD1 was conducted on steady state samples of the continuous cultivations in the presence and absence of benzoate. The transcriptomes were compared by analyzing the expression of genes via four pairwise comparisons. The genes involved in benzoate degradation (*ben, cat* and *pca* operons) comprised the majority of differentially expressed genes (Jiménez et al., 2002). When the strains were grown in the absence of benzoate, the transcriptomes of both strains were very similar except for a lower expression of *catA* and a higher expression of a gene encoding a hypothetical protein (PP_0032) in *P. putida* KT2440-JD1 (Table 2). When benzoate was present, the genes of the *ben* operon were highly induced in both strains. The genes of the *cat* operon were highly induced in *P. putida* KT2440, but their expression was very low in *P. putida* KT2440-JD1, showing that the induction of the *cat* operon is impaired in the mutant (Table 2). Furthermore, in the presence of benzoate the expression of two transporter genes (PP_3565 and PP_3566) was higher in *P. putida* KT2440-JD1 as compared to *P. putida* KT2440 (Table 2).

The results reveal that *P. putida* KT2440-JD1 accumulates cis, cis-muconate in the culture medium when benzoate is present due to the lack of induction of the *cat* operon. The *ben* operon contains a gene for a putative second catechol 1,2-dioxygenase (CatA2), which is probably responsible for the conversion of catechol to *cis, cis*-muconate in the mutant, since there were no other catechol 1,2-dioxygenase genes identified in the genome of in *P. putida* KT2440 (Nelson et al., 2002, Environ. Microbiol. 4,799-808).

The function of CatA2 has not yet been shown (Jiménez et al., 2002). CatA2 has 76 % amino acid identity with both CatA and the catechol 1,2-dioxygenase from *P. arvilla* C1, of which the three-dimensional structure has been elucidated. Based on the protein sequence alignment and visual structural comparison, the defined active sites of the three enzymes are 100% identical. To show that CatA2 is able to convert catechol to *cis, cis-* muconate, *catA2* was heterologously expressed in *E. coli* BLN21, and the enzyme was purified using the His-Tag. The results revealed that CatA2 is indeed functioning as a catechol 1,2-dioxygenase with a $K_m$ of 0.0010 mM catechol and a $IC_{50}$ of 0.1392 mM *cis,* cis-muconate at a concentration of 0.05 mM catechol. From these numbers a $K_i$ value of 0.0028 mM was calculated using the Cheng-Prusoff equation (Cheng and Prusoff, 1973). The $V_{max}$ *was* estimated to be 0.4 μM/min/mg.

The genes of the protocatechuate pathway (pcaBCD/JF) were still induced in *P. putida* KT2440-JD1, but the expression levels of the genes were approximately two times lower than those in the wildtype (Table 2). β-ketoadipate is known to induce the pca pathway and may be generated in the mutant by some remaining activity of the enzymes encoded by the cat operon. β-ketoadipate may also be formed by PcaB and PcaC, which convert β-carboxy-cis, cis-muconate and 7-carboxymuconolactone, respectively, but may have some activity for *cis,* cis-muconate and muconolactone as well, which could explain the observed $Y_{(P/S)}$ of 0.89 ±0.02 mol *cis, cis*-muconate/ mol benzoate.

*Proteomics of P. putida KT2440 and KT2440-JD1 in the presence and absence of benzoate*

Proteome analysis of *P. putida* KT2440 and KT2440-JD1 was conducted on steady state samples of the continuous cultivations. An average of 400 protein spots was displayed on each 2D gel, independent of the cultivation condition. When the strains were grown in the absence of benzoate, the proteomes of *P. putida* KT2440 and *P. putida* KT2440-JD1 were similar. In the presence of benzoate the only differences between *P. putida* KT2440 and KT2440-JD1 were the lower levels of CatA and CatB in the mutant, which confirmed on the proteomic level that the cat operon is no longer induced in *P. putida* KT2440-JD1. Other enzymes from the ortho-cleavage pathway that have been shown at the proteomic level (Cao et al., Appl. Microbiol. Biotechnol. 81,99-107 (2008); Cao and Loh, Biotechnol. Bioeng. 101,1297-1231 (2008); and Kim et al., Proteomics. 6,1301-1318 (2006)) were not identified in this study, which may be caused by a higher sensitivity of their analyses due to the use of longer IPG strips.

In both strains the same 15 proteins were differentially regulated in the presence and absence of benzoate (see Table 4). In the presence of benzoate, the expression levels of the chaperones GroEL, GroES and DnaK, which belong to the RpoH regulon family, were up-regulated. These proteins are induced by aromatics and mediate the proper folding of the proteins. The expression levels of *Catalase*/ *peroxidase* HPI, which is involved in the oxidative stress response, three outer membrane porin-like proteins (OprF, OmpA family protein and OprD) and a motility protein (FliC) were downregulated. *catA* and *catB* were the only genes that were differentially expressed both at the transcriptome and proteome level. The lack of similarity of the transcriptome and proteome may be caused by post-transcriptional regulatory systems, which determine the translation efficiency, the stability of the protein and its activity by modifications, and therefore influence the protein concentration in the cell. This illustrates the importance of multi-level studies.

*Nucleotide sequence of the cat operon of P. putida KT2440_JD1*

The analyses of the transcriptome and proteome revealed that the *cat operon* in *P. putida* KT2440-JD1 is no longer induced in the presence of benzoate. A 4,031 bp region containing the *cat operon* was sequenced and only one single point mutation was found in *catR.* This was at the first position of the $50^{th}$ codon, which changed Arg50 (CGC) to Cys50 (TGC). CatR belongs to the LysR-type transcriptional regulator (LTTR) proteins. An alignment of the amino acid sequences of seven different LTTR proteins showed that Arg50 is highly conserved in the winged helix-turn-helix motif of the DNA-binding region. The genes *catA* and *catBC are* divergently induced by the regulatory protein CatR in the presence of *cis, cis*-muconate. Apparently, the mutation in *catR* inhibited the expression of all genes of the cat operon. When *P. putida* KT2440-JD1 was complemented with wildtype *catR,* growth on benzoate as the single carbon source was restored. These results show unequivocally that the mutation in *catR* is responsible for the observed phenotype of *P. putida* KT2440-JD1.

Conclusions

*P. putida* KT2440-JD1, which was derived from the typestrain *P. putida* KT2440 after NTG mutagenesis and exposure to 3-FB, accumulates cis, cis-muconate from benzoate with a very high $q_{pm}$ of 2.09/($g_{DCW}$ × h) and $Y_{(P/S)}$ of 0.89 mol *(cis, cis*-muconate/ mol benzoate) under $\mu_{max}$ conditions and unlimited presence of benzoate. This $q_{pm}$ is at least eight times higher than the $q_{pm}$ values reported for other cis, cis-muconate-producing strains (Bang and Choi, 1995, J. Ferment. Bioeng. 79,381-383; Bang et al., 1996, Biotechnol. Bioprocess Eng. 1,36-40; Choi et al., 1997, J. Ferment. Bioeng. 84,70-76; Mizuno et al., 2003, Appl. Microbiol. Biotechnol. 28,20-25; Schmidt and Knackmuss, 1984; Wu et al., 2006, Biochem. Eng. J. 29,35-40), which shows that *P. putida* KT2440-JD1 is a very suitable biocatalyst for the production of *cis, cis*-muconate from benzoate.

*P. putida* KT2440-JD1 is the first cis, cis-muconate-accumulating mutant that was characterized at the genetic level. It contains a point mutation in *catR that* prevents the induction of the *cat operon* as a response to the presence of benzoate. The enzymes that are responsible for the conversion of benzoate to *cis, cis*-muconate in *P. putida* KT2440-JD1 are encoded on the *ben* operon, which contains the second catechol 1,2-dioxygenase-CatA2. CatA2 is 76% identical to other well-characterized catechol 1,2-dioxygenases, and has a fully conserved active site. It is the only catechol 1,2-dioxygenase-like gene that is highly expressed in the mutant, and its biochemical activity was determined.

**Table 1**

Growth and production of *cis, cis*-muconate by *P. putida* KT2440-JD1 in batch cultures with 10 mM of various substrates and 1 mM benzoate. The pre-cultures were grown on the same medium.

| growth substrate | maximum $q_{pm}$ g/($g_{DCW}$ × h) | $\mu_{max}$ $h^{-1}$ | $Y_{(P/S)}$ mol *cis, cis*-muconate/ mol benzoate | $Y_{(X/S)}$ $g_{DCW}$/ g growth substrate |
|---|---|---|---|---|
| Succinate | 0.5 | 1.0 | 0.68 | 0.29 |
| Citrate | 1.3 | 0.7 | 0.95 | 0.18 |
| Glucose | 2.0 | 0.7 | 0.89 | 0.30 |

**Table 2**

Transcriptomic data of genes that were differentially expressed with a fold change above 2 or below 0.5 and a threshold of 5 percent false positives. Comparison 1: *P. putida* KT2440-JD1 compared to *P. putida* KT2440 when grown in the absence of benzoate. Comparison 2: *P. putida* KT2440-JD1 compared to *P. putida* KT2440 when grown in the presence of benzoate. Comparison 3: *P. putida* KT2440 grown in the presence of benzoate compared to *P. putida* KT2440 grown in the absence of benzoate. Comparison 4: *P. putida* KT2440-JD1 grown in the presence of benzoate compared to *P. putida* KT2440-JD1 grown in the absence of benzoate.

| Comparison | Locus ID | Gene name | Product name | Fold change | Percent of false positives |
|---|---|---|---|---|---|
| 1 | PP_003 2 | | hypothetical protein | 2.28 | 0.00 |
| | PP_371 3 | *catA* | catechol 1,2-dioxygenase | 0.49 | 0.00 |
| 2 | PP_356 6 | | major facilitator family transporter | 3.87 | 0.00 |
| | PP_115 7 | | acetolactate synthase | 2.87 | 0.00 |
| | PP_312 3 | | 3-oxoacid CoA-transferase subunit B | 2.58 | 0.00 |
| | PP_265 2 | | hydratase/decarboxylase | 2.18 | 0.00 |
| | PP_001 7 | | transcriptional regulator MvaT, P16 subunit, putative | 2.30 | 0.00 |
| | PP_224 4 | | hypothetical protein | 2.13 | 0.00 |
| | PP_312 2 | | 3-oxoacid CoA-transferase subunit A | 2.08 | 0.00 |
| | PP_356 5 | | amino acid transporter LysE | 2.04 | 0.00 |
| | PP_446 6 | | TauD/TfdA family dioxygenase | 2.02 | 0.00 |
| | PP_137 7 | *pcaF* | beta-ketoadipyl CoA thiolase | 0.44 | 0.02 |
| | PP_138 0 | *pcaD* | 3-oxoadipate enol-lactonase | 0.42 | 0.01 |
| | PP_395 1 | *pcaI* | 3-oxoadipate CoA-transferase, subunit A | 0.40 | 0.02 |
| | PP_395 2 | *pcaJ* | 3-oxoadipate CoA-transferase, subunit B | 0.40 | 0.02 |
| | PP_371 2 | | hypothetical protein | 0.41 | 0.02 |
| | PP_255 3 | | major facilitator family transporter | 0.36 | 0.01 |

(continued)

| Transcriptomic data of genes that were differentially expressed with a fold change above 2 or below 0.5 and a threshold of 5 percent false positives. Comparison 1: *P. putida* KT2440-JD1 compared to *P. putida* KT2440 when grown in the absence of benzoate. Comparison 2: *P. putida* KT2440-JD1 compared to *P. putida* KT2440 when grown in the presence of benzoate. Comparison 3: *P. putida* KT2440 grown in the presence of benzoate compared to *P. putida* KT2440 grown in the absence of benzoate. Comparison 4: *P. putida* KT2440-JD1 grown in the presence of benzoate compared to *P. putida* KT2440-JD1 grown in the absence of benzoate. | | | | | |
|---|---|---|---|---|---|
| Comparison | Locus ID | Gene name | Product name | Fold change | Percent of false positives |
| | PP_264 3 | | methyl-accepting chemotaxis sensory transducer | 0.28 | 0.00 |
| | PP 371 5 | *catB* | muconate and chloromuconate cycloisomerase | 0.11 | 0.00 |
| | PP_371 4 | *catC* | muconolactone isomerase | 0.03 | 0.00 |
| | PP_371 3 | *catA* | catechol 1,2-dioxygenase | 0.03 | 0.00 |
| 3 | PP_371 4 | *catC* | muconolactone isomerase | 16.29 | 0.00 |
| | PP_371 3 | *catA* | catechol 1,2-dioxygenase | 15.43 | 0.00 |
| | PP_316 2 | *benB* | muconate and chloromuconate cycloisomerase | 14.83 | 0.00 |
| | PP_316 6 | *ca-tA2* | catechol 1,2-dioxygenase | 15.39 | 0.00 |
| | PP_316 3 | *benC* | oxidoreductase FAD/NAD(P)-binding domain protein | 14.21 | 0.00 |
| | PP_316 5 | *benK* | major facilitator transporter | 13.76 | 0.00 |
| | PP_316 1 | *benA* | benzoate dioxygenase, alpha subunit | 12.50 | 0.00 |
| | PP_316 4 | *benD* | 1,6-dihydroxycyclohexa-2,4-diene-1-carboxylate dehydrogenase | 11.91 | 0.00 |
| | PP_316 8 | *benF* | benzoate-specific porin | 11.01 | 0.01 |
| | PP_316 7 | *benE-2* | benzoate transporter | 9.63 | 0.00 |
| | PP_371 5 | *catB* | muconate and chloromuconate cycloisomerase | 8.44 | 0.01 |
| | PP_395 1 | *pcaI* | 3-oxoadipate CoA-transferase, subunit A | 5.54 | 0.01 |
| | PP_395 2 | *pcaJ* | 3-oxoadipate CoA-transferase, subunit B | 4.71 | 0.01 |
| | PP_264 3 | | methyl-accepting chemotaxis sensory transducer | 4.52 | 0.01 |
| | PP_137 7 | *pcaF* | beta-ketoadipyl CoA thiolase | 4.51 | 0.01 |
| | PP_138 0 | *pcaD* | 3-oxoadipate enol-lactonase | 3.74 | 0.02 |
| | PP_316 9 | | membrane-bound metal-dependent hydrolase | 3.62 | 0.02 |
| | PP_137 6 | *pcaK* | benzoate transport | 3.32 | 0.02 |
| | PP_138 3 | | BenF-like porin | 3.22 | 0.02 |
| | PP_255 3 | | major facilitator family transporter | 3.08 | 0.02 |
| | PP_138 1 | *pcaC* | 4-carboxymuconolactone decarboxylase | 2.75 | 0.03 |
| | PP_371 2 | | hypothetical protein | 2.66 | 0.02 |

(continued)

| Transcriptomic data of genes that were differentially expressed with a fold change above 2 or below 0.5 and a threshold of 5 percent false positives. Comparison 1: *P. putida* KT2440-JD1 compared to *P. putida* KT2440 when grown in the absence of benzoate. Comparison 2: *P. putida* KT2440-JD1 compared to *P. putida* KT2440 when grown in the presence of benzoate. Comparison 3: *P. putida* KT2440 grown in the presence of benzoate compared to *P. putida* KT2440 grown in the absence of benzoate. Comparison 4: *P. putida* KT2440-JD1 grown in the presence of benzoate compared to *P. putida* KT2440-JD1 grown in the absence of benzoate. | | | | | |
|---|---|---|---|---|---|
| Comparison | Locus ID | Gene name | Product name | Fold change | Percent of false positives |
| | PP_367 1 | | aldo/keto reductase family oxidoreductase | 2.49 | 0.02 |
| | PP_137 9 | *pcaB* | 3-carboxy-cis,cis-muconate cycloisomerase | 2.31 | 0.03 |
| | PP_315 9 | *benR* | AraC family transcriptional regulator | 2.18 | 0.03 |
| | PP_174 2 | | hypothetical protein | 0.34 | 0.04 |
| 4 | PP_316 6 | *ca-tA2* | catechol 1,2-dioxygenase | 15.34 | 0.00 |
| | PP_316 5 | *benK* | major facilitator transporter | 13.01 | 0.00 |
| | PP_316 3 | *benC* | oxidoreductase FAD/NAD(P)-binding domain protein | 12.35 | 0.00 |
| | PP_316 2 | *benB* | muconate and chloromuconate cycloisomerase | 12.54 | 0.00 |
| | PP_316 1 | *benA* | benzoate dioxygenase, alpha subunit | 11.70 | 0.00 |
| | PP_316 4 | *benD* | 1,6-dihydroxycyclohexa-2,4-diene-1-carboxylate dehydrogenase | 9.25 | 0.00 |
| | PP_316 8 | *benF* | benzoate-specific porin | 8.93 | 0.00 |
| | PP_316 7 | *benE-2* | benzoate transporter | 7.75 | 0.00 |
| | PP_356 6 | | major facilitator family transporter | 4.09 | 0.00 |
| | PP_115 7 | | acetolactate synthase | 2.97 | 0.00 |
| | PP_316 9 | | membrane-bound metal-dependent hydrolase | 2.72 | 0.01 |
| | PP_265 2 | | hydratase/decarboxylase | 2.44 | 0.01 |
| | PP_312 3 | | 3-oxoacid CoA-transferase subunit B | 2.42 | 0.01 |
| | PP_395 1 | *pcaI* | 3-oxoadipate CoA-transferase, subunit A | 2.35 | 0.01 |
| | PP_137 7 | *pcaF* | beta-ketoadipyl CoA thiolase | 2.22 | 0.02 |
| | PP_137 6 | *pcaK* | benzoate transport | 2.06 | 0.02 |
| | PP_001 7 | | transcriptional regulator MvaT, P16 subunit, putative | 2.03 | 0.03 |
| | PP_312 2 | | 3-oxoacid CoA-transferase subunit A | 2.01 | 0.03 |
| | PP_200 6 | | hypothetical protein | 0.49 | 0.01 |
| | PP_003 2 | | hypothetical protein | 0.48 | 0.03 |

**Table 4.**

Proteomics data of proteins that were differentially regulated with a fold change above 1.5 or below 0.7 and a MOWSE score higher than 50 %. Comparison 1: *P. putida* KT2440-JD1 compared to *P. putida* KT2440 when grown in the absence of benzoate. Comparison 2: *P. putida* KT2440-JD1 compared to P. *putida* KT2440 when grown in the presence of benzoate. Comparison 3: P. *putida* KT2440 grown in the presence of benzoate compared to *P. putida* KT2440 grown in the absence of benzoate. Comparison 4: *P. putida* KT2440-JD1 grown in the presence of benzoate compared to *P. putida* KT2440-JD1 grown in the absence of benzoate.

| Comparison | Locus ID | Protein name | Protein name | Fold change | MOWSE score |
|---|---|---|---|---|---|
| 1 | No differences | | | | |
| 2 | PP_3713 | CatA | catechol 1,2-dioxygenase | 0.5 | 85 |
| | PP_3715 | CatB | muconate and chloromuconate cycloisomerase | 0.4 | 92 |
| 3/4 | PP_1361 | GroEL | chaperonin GroEL | 2.0 | 102 |
| | PP_4849 | | dnaK protein, putative | 1.8 | 163 |
| | PP_1360 | GroES | co-chaperonin GroES | 1.8 | 57 |
| | PP_0469 | RplF | 50S ribosomal protein L6 | 1.5 | 115 |
| | PP_0454 | RplC | 50S ribosomal protein L3 | 1.5 | 131 |
| | PP_5393 | | heavy metal transport/ detoxification protein | 1.5 | 57 |
| | PP_0466 | RplE | 50S ribosomal protein L5 | 0.7 | 144 |
| | PP_2089 | OprF | OmpF family protein | 0.7 | 146 |
| | PP_1001 | ArcA | arginine deiminase | 0.7 | 53 |
| | PP_1087 | | OmpA family outer membrane protein | 0.7 | 97 |
| | PP_1206 | OprD | outer membrane porin | 0.7 | 128 |
| | PP_4378 | FliC | flagellin FliC | 0.7 | 147 |
| | PP_1661 | | dehydrogenase subunit, putative | 0.6 | 120 |
| | PP_0766 | | hypothetical protein | 0.6 | 57 |
| | PP_3668 | | catalase/ peroxidase HPI | 0.6 | 82 |

## Claims

1. An engineered host cell for the production of cis, cis-muconic acid from catechol which host cell comprises a foreign gene of SEQ ID No. 1 or derivatives thereof encoding for a polypeptide of SEQ ID No. 2 having a catechol 1,2-dioxygenase activity.

2. The engineered host cell according to claim 1 which is a microorganism, in particular, a bacterial strain.

3. The engineered host cell according to claim 1 which is a eukaryotic cell, in particular, a yeast cell.

4. The engineered host cell according to any one of the preceding claims wherein the endogenous gene encoding for the catechol 1,2-dioxygenase and/or the catB gene of the host cell is silenced, preferably knocked-down or knocked-out, or deleted from e.g. the chromosome of the host cell.

5. The engineered host cell according to any one of the preceding claims comprising additionally foreign genes encoding for polypeptides being enzymes active in the metabolic pathway of from benzoate to cis, cis-muconic acid, in particular said genes are selected from benA, benB, benC, or benD.

6. A pseudomonas strain wherein the catB gene is silenced, preferably knocked-down or knocked-out, or deleted from the chromosome.

7. The pseudomonas strain according to claim 6 wherein the catB gene is a gene of SEQ ID No. 5 or derivatives thereof encoding for a polypeptide of SEQ ID No. 6.

8. The pseudomonas strain according to claim 6 or 7 wherein the pseudomonas strain is a mutant pseudomonas strain being catR deficient.

9. A method for the production of cis, cis-muconic acid comprising the step of culturing an engineered host cell according to any one of claims 1 to 5 or a pseudomonas strain according to any one of claims 6 to 8 under culture conditions in which the cultured host cell or pseudomonas strain produces cis, cis-muconic acid.

10. A method according to claim 9 wherein the culture medium comprises introduction of benzoate, and/or glucose.

11. The method according to claim 9 or 10 wherein benzoate is introduced in an amount of at least 1 mmol.

12. A method for the production of adipic acid comprising the production of cis, cis-muconic acid according to the method of any one of claim 9 to 11 and, further, hydrogenation of the cis, cis-muconic acid for conversion to adipic acid.

**13.** The method according to claim 13 comprising the step of enriching, preferably, isolating cis, cis-muconic acid from the supernatant and/or biomass of host cells obtained according to the method of any one of claims 9 to 11.

**14.** A method for the generation of host cells for the production of cis, cis-muconic acid from catechol comprising the step of introducing a foreign gene of SEQ ID No. 1 or derivatives thereof encoding for a polypeptide of SEQ ID No. 2 having a catechol 1,2-dioxygenase activity, into the host cell.

**15.** The method according to claim 14 wherein the foreign gene is introduced into the genome of the host cell.

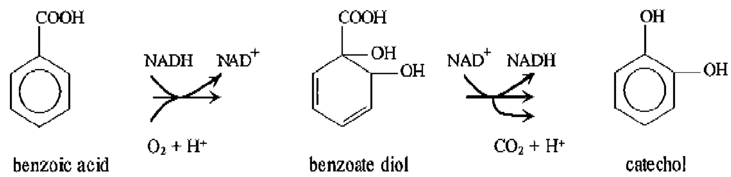


benzoic acid | benzoate diol | catechol

*benABC*
benzoate dioxygenase

*benD*
benzoate diol dehydrogenase

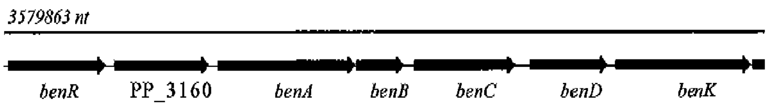


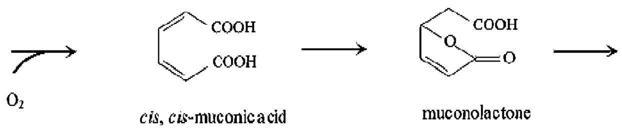


*cis, cis*-muconic acid | muconolactone

*catA2/ catA*
catechol 1,2-dioxygenase

*catB*
muconate lactonizing enzyme

*catC*
muconolactone isomerase

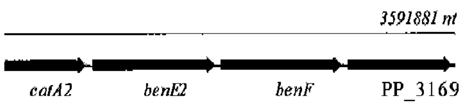


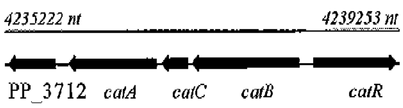


Figure 1 (1)

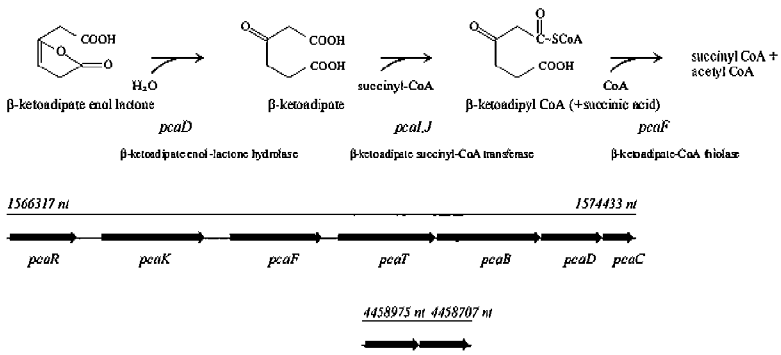
H₂O
succinyl-CoA
CoA
succinyl CoA +
acetyl CoA
β-ketoadipate enol lactone
β-ketoadipate
β-ketoadipyl CoA (+succinic acid)
pcaD
pcaIJ
pcaF
β-ketoadipate enol-lactone hydrolase
β-ketoadipate succinyl-CoA transferase
β-ketoadipate-CoA thiolase
1566317 nt
1574433 nt
pcaR
pcaK
pcaF
pcaT
pcaB
pcaD
pcaC
4458975 nt
4458707 nt

Figure 1(2)

Europäisches Patentamt
European Patent Office
Office européen des brevets

# EUROPEAN SEARCH REPORT

Application Number
EP 11 17 8713

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "2nd International PhD Symposium", Helmholtz International Research School for Infection Biology (HIRSIB) , 11 December 2008 (2008-12-11), page 41PP, XP002667137, Retrieved from the Internet: URL:http://www.hzigradschool.de/fileadmin/user_upload/_archiv/Gradschool/BoA.pdf [retrieved on 2012-01-13] * page T11 * | 1-5,9-15 | INV. C12N9/02 C12N9/90 C12P7/46 |
| X,D | JIMENEZ J I ET AL: "Genomic analysis of the aromatic catabolic pathways from Pseudomonas putida KT2440", ENVIRONMENTAL MICROBIOLOGY, vol. 4, no. 12, 1 December 2002 (2002-12-01), pages 824-841, XP003003933, BLACKWELL SCIENCE, OXFORD, GB ISSN: 1462-2912, DOI: 10.1046/J.1462-2920.2002.00370.X * page 828; table 2 * | 1-3,5, 9-11, 13-15 | |
| X | NELSON K E ET AL: "Complete genome sequence and comparative analysis of the metabolically versatile pseudomonas putida KT2440", ENVIRONMENTAL MICROBIOLOGY, vol. 4, no. 12, 1 December 2002 (2002-12-01), pages 799-808, XP002968819, BLACKWELL SCIENCE, OXFORD, GB ISSN: 1462-2912, DOI: 10.1046/J.1462-2920.2002.00366.X * table 1 * | 1-3,5, 9-11, 13-15 | |
| | -/-- | | |

TECHNICAL FIELDS SEARCHED (IPC)
C12N
C12P

~~The present search report has been drawn up for all claims~~

2

| Place of search | Date of completion of the search | Examiner |
|---|---|---|
| The Hague | 16 January 2012 | Lejeune, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 17 8713

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KIM B J ET AL: "Enhancement of cis,cis-muconate productivity by overexpression of catechol 1,2-dioxygenase in Pseudomonas putida BCM114", BIOTECHNOL BIOPROCESS ENG, vol. 3, 1998, pages 112-114, XP002667138, * the whole document * ----- | 1-5,9-15 | |
| A,D | DATABASE UniProt [Online] 1 June 2003 (2003-06-01), "SubName: Full=Catechol 1,2-dioxygenase;", XP002667139, retrieved from EBI accession no. UNIPROT:Q88I35 Database accession no. Q88I35 * sequence * ----- | 1-5 | |
| | | | TECHNICAL FIELDS SEARCHED (IPC) |

~~The present search report has been drawn up for all claims~~

| Place of search | Date of completion of the search | Examiner |
|---|---|---|
| The Hague | 16 January 2012 | Lejeune, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

Application Number

EP 11 17 8713

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-3, 5, 14, 15(completely); 4, 9-13(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION
# SHEET B

Application Number
EP 11 17 8713

The Search Division considers that the present European patent application does not comply with the requirements of unity of invention and relates to several inventions or groups of inventions, namely:

1. claims: 1-3, 5, 14, 15(completely); 4, 9-13(partially)

   Host cell for the production of cis,cis-muconic acid comprising a foreign gene of SEQ ID NO 1 encoding for a polypeptide having catechol 1,2-dioxygenase activity; use thereof in a method to produce cis,cis-muconic acid.

   ---

2. claims: 6-8(completely); 4, 9-13(partially)

   Pseudomonas strain wherin the catB gene is silenced; use thereof in a method to produce cis,cis-muconic acid

   ---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 518188 A


### Non-patent literature cited in the description


- **NELSON et al.** *Environ. Microbiol.,* 2002, vol. 4, 799-808
- **JIMÉNEZ et al.** *Environ. Microbiol.,* 2002, vol. 4, 824-841
- **KIM et al.** *Biotechnol. Bioprocess Eng.,* 1988, vol. 3, 112-114
- **SCHMIDT, E. ; KNACKMUSS, HJ.** *Apl. Microbiol. Biotechnol.,* 1984, vol. 20, 351-355
- **BANG S.G. ; CHOI S.Y.** *J. Ferment. Bioeng.,* 1995, vol. 79, 381-383
- **MIZUNO S.** *Apl. Microbiol. Biotechnol,* 1988, vol. 28, 20-25
- **WU C.M.** *Biochem. Eng. J.,* 2006, vol. 29, 35-40
- **JIMÉNEZ.** *Environ. Microbiol.,* 2002, vol. 4, 824-841
- **SCHMIDT E ; KNACKMUSS HJ.** *Appl Microbiol Biotechnol,* 1984, vol. 20, 351-355
- **BANG SG ; CHOI CY.** *J Ferment Bioeng,* 1995, vol. 79, 381-383
- **CHOI WJ ; LEE EY ; CHO MH ; CHOI CY.** *J Ferment Bioeng,* 1997, vol. 84, 70-76
- **MIZUNO S ; YOSHIKAWA N ; SCHI M ; MIKAWA T ; IMADA Y.** *Appl Microbiol Biotechnol,* 1988, vol. 28, 20-25
- **HARTMANS et al.** *Appl. Environ. Microbiol.,* 1989, vol. 55, 2850-2855
- **JEFFREY et al.** *J. Bacteriol.,* 1992, vol. 174, 4986-4996
- **ADELBERG et al.** *Biochem. Biophys. Res. Commun.,* 1965, vol. 18, 788-795
- **BALLERSTEDT et al.** *Appl. Microbiol. Biotechnol.,* 2007, vol. 75, 1133-1142
- **GENTLEMAN et al.** *Genome Biol.,* 2004, vol. 5, R80
- **WU et al.** *Biochem. Eng. J.,* 2004, vol. 29, 35-40
- **BREITLING et al.** *FEBS Lett.,* 2004, vol. 573, 83-92
- **DUDOIT et al.** *Stat. Sci.,* 2003, vol. 18, 71-103
- **WIJTE et al.** *J. Proteome. Res.,* 2006, vol. 5, 2033-2038
- **KRIEG et al.** *Biotechniques,* 2003, vol. 35, 376-378
- **HAVLIS et al.** *Anal. Chem.,* 2003, vol. 75, 1300-1306
- **THOMPSON et al.** Curr. Protoc. Bioinformatics. 2002
- **EARHART et al.** *Biochem. Biophys. Res. Commun.,* 2005, vol. 338, 198-205
- **EMSLEY ; COWTAN.** *Acta Crystallogr. D Biol Crystallogr.,* 2004, vol. 60, 2126-2132
- **SCHMIDT ; KNACKMUSS.** *Appl. Microbiol. Biotechnol.,* 1984, vol. 20, 351-355
- **BANG ; CHOI.** *J. Ferment. Bioeng.,* 1995, vol. 79, 381-383
- **CAO et al.** *Appl. Microbiol. Biotechnol.,* 2008, vol. 81, 99-107
- **CAO ; LOH.** *Biotechnol. Bioeng.,* 2008, vol. 101, 1297-1231
- **KIM et al.** *Proteomics,* 2006, vol. 6, 1301-1318
- **BANG et al.** *Biotechnol. Bioprocess Eng.,* 1996, vol. 1, 36-40
- **CHOI et al.** *J. Ferment. Bioeng.,* 1997, vol. 84, 70-76
- **MIZUNO et al.** *Appl. Microbiol. Biotechnol.,* 2003, vol. 28, 20-25
- **WU et al.** *Biochem. Eng. J.,* 2006, vol. 29, 35-40